# EUROPEAN PATENT APPLICATION

(11) **EP 4 382 131 A1**
(43) Date of publication of application: **12.06.2024**
(21) Application number: 22212011.5
(22) Date of filing: 07.12.2022
(51) Int. Cl.: A61K 47/69, A61K 39/00, A61P 35/00

(54) **BEAD BOUND ANTICD28 ANTIBODY FOR THE TREATMENT OF MALIGNANT MELANOMA**

(71) Applicant: Julius-Maximilians-Universität Würzburg, 97070 Würzburg (DE); Fraunhofer-Gesellschaft zur Förderung der angewandten Forschung e.V., 80686 München (DE)
(72) Inventor: Schilling, Bastian, 97082 Würzburg (DE); Beyersdorf, Niklas, 97076 Würzburg (DE); Kerkau, Thomas, 97070 Würzburg (DE); Groeber-Becker, Florian, 97082 Würzburg (DE)
(74) Representative: Engelhard, Markus

(57) **Abstract**

The present invention relates to solid microbeads having an anti-CD28 monoclonal antibody immobilized thereto for use in the local treatment of malignant melanoma, wherein the anti-CD28 monoclonal antibody binds to an epitope on CD28 outside the C"D loop. The present invention further relates to methods for the local treatment of malignant melanoma comprising the administration of said microbeads.

## Description

The present invention relates to solid microbeads having an anti-CD28 monoclonal antibody immobilized thereto for use in the local treatment of malignant melanoma, wherein the anti-CD28 monoclonal antibody binds to an epitope on CD28 outside the C"D loop. The present invention further relates to methods for the local treatment of malignant melanoma comprising the administration of said microbeads.

### BACKGROUND OF THE INVENTION

Melanoma is a severe and lethal malignant transformation of skin cells that undergo uncontrolled proliferation. Melanoma shows a high incidence rate and genomic alterations with a high tendency to metastasize (up to 20% of patients) (see Kang et al., 2020; WHO. Melanoma of skin Source: Globocan 2020; WHO. Cancer Today 2020.) Several immunotherapies have been in use to treat melanoma but they are still of limited efficacy and lack durability (Schummer *et al.,* 2020; Wu *et al.,* 2019; Ribas and Wolchok, 2018; Nowicki *et al.,* 2018; Yeung *et al.,* 2020; Wei *et al*., 2018; Jiang *et al*., 2021).

The elimination of malignant cells requires the strong and accurately targeted cytotoxic mechanisms primarily of CD8⁺ T cells. Upon target cell identification, i.e. after recognition of their specific surface-antigens presented on major histocompatibility complexes (MHC), CD8⁺ T cells start releasing cytotoxic proteins (i.e. perforin and Granzyme B). CD8⁺ T cells also release cytokines like IFNγ and TNF-α which contribute to their cytotoxic activity (Castro *et al.,* 2018; Platanias 2005).

In addition to the cytotoxic activity of CD8⁺ T cells in response to MHC class I antigen expression, the involvement of tumor-reactive CD4⁺ T cells improves the anti-tumor efficacy of CD8⁺ T cells especially upon MHC class I and II Ag expression (see e.g. Alspach *et al.,* 2019). Tumor-reactive CD4⁺ T cells were found to exert their helper function in the priming of MHC class I-restricted CD8⁺ T cells driving their maturation into CTL (reviewed in Smith-Garvin *et al.,* 2009). Moreover, CD4⁺ T cells promote anti-tumor cytotoxicity by killing the target cells in an MHC class II-dependent or -independent manner (see e.g. Rosskopf *et al.,* 2019).

Naive T cells require two signals for full activation (Chen and Flies, 2013): Signal 1 is generated in the T cell after the T cell receptor binds and recognizes a peptide bound to MHC molecules. Signal 2 stems from interaction of the CD28 receptor expressed by T cells with B7 molecules expressed by antigen-presenting cells (APC). Signal 2 indicates to the T cell that the APC has sensed 'danger', i.e. that there is a pathogen/ something harmful in the body.

Monoclonal antibody (mAb) fragments against the CD28 costimulatory receptor have been developed to block interaction of CD28 with its ligands and, thus, suppress graft rejection (reviewed in Adams *et al.,* 2016) as well as autoimmune responses such as in rheumatoid arthritis (reviewed in Vanhove *et al.,* 2017). As intact antibodies these mAbs bind to or close to the MYPPPY motif of CD28 to which also the B7 molecules bind. Functionally, the mAbs are capable of mimicking ligation of CD28 by B7 molecules, i.e. these antibodies provide costimulation to T cells receiving a T cell receptor signal. They are, thus, referred to as 'conventional' anti-CD28 antibodies (reviewed in Poirier *et al.,* 2012).

Moreover, so-called superagonistic CD28 stimulation induced by anti-CD28 mAb binding to the C"D loop of CD28 and leading to full T cell activation in the absence of T cell receptor complex stimulation has been exploited for drug development. The anti-human CD28 superagonist (CD28-SA) TAB08/ TGN1412, however, unexpectedly induced a cytokine release syndrome in healthy volunteers during a first-in-man study (Suntharalingam *et al*., 2009; Eastwood *et al*., 2010). This was in contrast to animal models of autoimmunity in which CD28-SA application had mediated protection without induction of toxicity (see e.g. Suntharalingam *et al.,* 2009; Römer *et al.,* 2011; Beyersdorf, Gaupp *et al.,* 2005). The difference between the animal models used and the human volunteers was that the animals lacked a true memory T cell compartment so that in the animals CD28-SA application strongly activated CD4⁺ Foxp3⁺ regulatory T cells, but not memory T cells secreting pro-inflammatory cytokines (Eastwood *et al.,* 2010).

There is a need in the art for improved means and methods for the treatment of metastatic disease, which particularly allow highly potent local immunotherapy.

### SUMMARY OF THE INVENTION

According to the present invention this object is solved by a solid microbead having an anti-CD28 monoclonal antibody immobilized thereto for use in the local treatment of malignant melanoma,
wherein the anti-CD28 monoclonal antibody binds to an epitope on CD28 outside the C"D loop.

According to the present invention this object is solved by a method for the local treatment of malignant melanoma comprising the step of
administering a therapeutically effective amount of solid microbeads as defined in the present invention, or
administering a pharmaceutical composition comprising the solid microbeads as defined in the present invention.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "0.5 to 10" should be interpreted to include not only the explicitly recited values of 0.5 to 10, but also include individual values and sub-ranges within the indicated range. Thus, included in this numerical range are individual values such as 0.5, 1, 1.5, 2, 2.5, 3, 4, 5 .... 8, 8.5, 9, 9.5, 10 and sub-ranges such as from 2 to 8, 4 to 5, etc. This same principle applies to ranges reciting only one numerical value, such as "less than 1 mm". Furthermore, such an interpretation should apply regardless of the breadth of the range or the characteristics being described.

### Anti-CD28 microbeads

As outlined above, the present invention provides a solid microbead having an anti-CD28 monoclonal antibody immobilized thereto for use in the local treatment of malignant melanoma.

### - Anti-CD28 antibody

According to the invention, the anti-CD28 monoclonal antibody is an anti-human CD28 monoclonal antibody.

According to the invention, the anti-CD28 monoclonal antibody binds to an epitope on CD28 outside the C"D loop.

Preferably, the anti-CD28 monoclonal antibody is a so called "conventional" anti-CD28 monoclonal antibody.

Examples for conventional anti-CD28 antibodies are clones 15E8, CD28.2 and L293.

15E8 or clone 15E8 is commercially available from Sigma Aldrich (catalog no. CBL517) and Miltenyi Biotec (catalog no. 130-093-375). 15E8 has been described in Van Lier *et al.* (1989), Pohl *et al.* (1993) and Hombach *et al.* (2009).

CD28.2 is commercially available from BD Biosciences (catalog no. 555725), Santa Cruz Biotechnology (catalog no. SC-19655). CD28.2 has been first described by Nunes *et al.* (1993).

L293 is commercially available from Fisher Scientific (catalog no. BDB340975) or BD Biosciences (catalog no. 348040). L293 has been first described by Azuma *et al.* (1992).

More preferably, the anti-CD28 monoclonal antibody is not a super-agonistic anti-CD28 monoclonal antibody.

So-called "conventional" anti-CD28 monoclonal antibodies can further be defined as follows: "Conventional" anti-CD28 mAb's can be identified by analyzing their capacity to block binding of a soluble ligand for CD28, CD80-lg, to CD28 expressed by CD4+ T cells. Thus, a conventional anti-CD28 mAb is characterized by binding an epitope on the CD28 molecule close to the binding site of the ligands of CD28, CD80 and CD86, and therefore outside the so-called C"D loop, which has been identified as a key feature of conventional anti-CD28 mAb (see Luhder *et al.,* 2003).

In order to clearly distinguish conventional from so-called superagonistic anti-CD28 antibodies, the method published by Römer *et al.* (2011) which increases the sensitivity of human T cells isolated from peripheral blood towards activating reagents and, thus, has the potential to reveal superagonistic activity of anti-CD28 mAb, may be used. This is also called the RESTORE protocol. Further, conventional anti-CD28 antibodies are unable to induce proliferation of T cells when added as the sole active reagent to single cell suspensions of splenocytes or lymph node cells.

Conventional anti-CD28 mAb can be distinguished from those having superagonistic activity in that binding of the (conventional) anti-CD28 mAb to an epitope on the CD28 molecule close to the ligand binding site precludes superagonistic activity of an anti-CD28 mAb.

A superagonist further can be defined as a type of agonist that is capable of producing a maximal response greater than the endogenous agonist for the target receptor, and thus has an efficacy of far more than 100% (for example 200, 500 or 1000%) (see Dennehy *et al.,* 2007) and may even induce a qualitatively different response in comparison to the endogenous agonist. Further information on how to distinguish conventional from superagonistic anti-CD28 mAb can be found in (Beyersdorf *et al.,* 2005) which is incorporated herein by reference. As noted therein, superagonistic anti-CD28 antibodies bind to a lateral, membrane-proximal loop of the molecule (C"D loop).

The amino acid sequence of human CD28 is known under the accession no. NM-006139. The C"D loop of CD28 comprises amino acids 52 to 66 of the above CD28 sequence (see also Ostrov *et al.,* 2000). The term C"D loop as used herein shall also comprise any partial sequences thereof.

The term "antibody" as used herein refers to intact antibodies as well as antibody fragments, which have the ability to selectively bind to an epitope. Such fragments include, without limitations, Fab, F(ab')2, Fv and scFv antibody fragments. The term "epitope" means any antigen determinant of an antigen, to which the paratope of an antibody can bind. Epitope determinants usually consist of chemically active surface groups of molecules (e.g. amino acid or sugar residues) and usually display a three-dimensional structure as well as specific physical properties. The term antibody also comprises chimeric and humanized antibodies.

The production of monoclonal antibodies, such as the above anti-CD28 monoclonal antibodies, is well known in the art. Examples include the hybridoma method (Kohler and Milstein, 1975; Coligan *et al*., 1992; Harlow *et al*., 1988).), the trioma technique, the human B-cell hybridoma technique (Kozbor *et al.,* 1983), and the EBV-hybridoma technique to produce monoclonal antibodies (Cole *et al*., 1985).

### - Microbeads

According to the invention, the anti- CD28 monoclonal antibody is immobilized on solid microbeads.

A "microbead" as used herein refers to a solid bead having a diameter of less than 100 µm, preferably less than 50 µm.

Preferably, the microbeads have a diameter in the range of about 0.5 µm to about 10 µm, preferably 4 to 5 µm, such as 4.5 µm.

The microbeads are preferably magnetic beads.

Magnetic beads suitable for the use in the present invention are of a variety of compositions including, but not limited to, fine grains of iron oxides dispersed throughout the interior of a polymer particle, silanized beads of magnetic iron oxides, and silanized beads of magnetic porous glass. Polymer compositions of magnetic polymer particles include, for example, polystyrene, cellulose, and latex. Commercially available magnetic beads are available with surfaces modified so as to allow for direct or indirect attachment of molecules, such as an antibody, to the bead surface. Surface modifications of such beads include, but are not limited to, immobilized antibodies such as, without limitation, secondary antibodies; immobilized protein A, immobilized protein G, immobilized streptavidin, immobilized avidin, immobilized biotin, immobilized oligo (dT), and having end groups such as, without limitation, -COOH, - NH2, -OH, epoxy-, tosyl-activated, hydrazide, and glyceryl.

A preferred group of microbeads for use in the present invention are magnetic microbeads, preferably superparamagnetic polystyrene microbeads.

In a preferred embodiment of the invention, the anti-CD28 monoclonal antibody is indirectly immobilized to the microbeads, preferably via another antibody.

For example, the microbeads are coated with a monoclonal anti-mouse IgG antibody (such as those commercially available under the tradename Dynabeads^{®} Pan Mouse IgG manufactured by Dynal^{®} Biotech).

Dynabeads^{®} Pan Mouse IgG are uniform, superparamagnetic polystyrene beads (having a 4.5 µm diameter) coated with a monoclonal human anti-mouse IgG antibody. The antibody coated onto Dynabeads^{®} recognizes all mouse IgG subclasses and is Fc-specific. The use of an indirect immobilization has the following advantages: a uniform binding of the anti-CD28 monoclonal mAbs is guaranteed; further, indirect binding of the anti-CD28 mAb to the microbead via a linker such as the human anti-mouse IgG-Fc mAb increases the steric flexibility of the immobilized anti-CD28 mAb, which should in turn facilitate interaction of the mAb with the CD28 molecule. However, the present invention is not restricted to an antibody as a linker.

In a preferred embodiment, the anti-CD28 monoclonal antibody is the only active ligand or only active principle immobilized onto the microbeads.

That is to say, the solid microbeads incorporate anti-CD28 antibodies as the only active principle whereas it is not excluded that antibodies used for indirect immobilization are bound to the microbead as well.

Preferably, there is no further anti-CD antibody immobilized on the microbead.

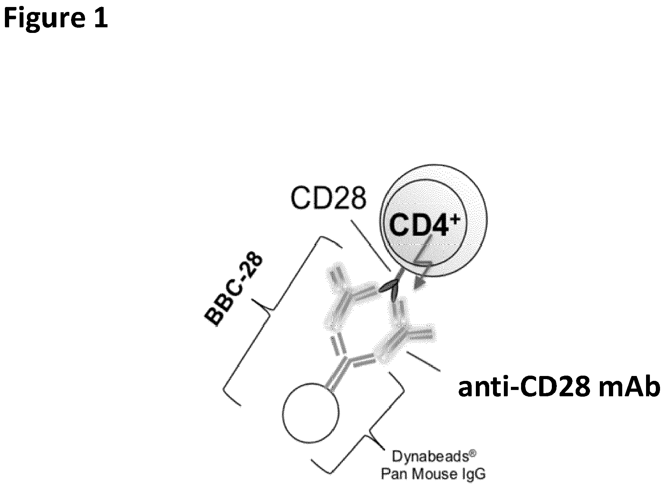

For example, the microbead may be coated with an anti-mouse Ig monoclonal antibody to which the conventional anti-CD28 monoclonal antibody is bound (see the illustration in Figure 1). Thus, a solid microbead as used according to the invention may consist of (or consist essentially of) a solid microbead, an anti-mouse Ig monoclonal antibody and an anti-CD28 monoclonal antibody.

The solid microbeads having an anti-CD28 monoclonal antibody immobilized thereto of the present invention are also called "BBC-28" herein.

BBC-28 are conventional, i.e. non-superagonistic, anti-CD28 mAb immobilized on superparamagnetic polystyrene beads, i.e.Dynabeads^{™} Pan Mouse IgG (Invitrogen). Dynabeads^{™} (DB) are coated with monoclonal human anti-mouse IgG antibodies specific for the Fc part of the mouse anti-human CD28 mAb, as described by the inventors in WO 2015/091919 A1, which is incorporated herein by reference. These human anti-mouse IgG antibodies allow indirect immobilization, uniform binding and steric flexibility of the anti-CD28 mAb to facilitate its interaction with the CD28 molecule.

BBC-28 have been shown to provide for an *in vitro* method for CD4⁺ memory T cell expansion which does not require TCR engagement (left free for Ag recognition) and leads to the desired stimulation of CD4⁺T cells and, CD4⁺ T cell-dependently, CD8⁺ T cells, while avoiding preferential activation and expansion of CD4⁺ Foxp3⁺ regulatory T cells seen with CD28-SA under certain conditions, as described by the inventors in WO 2015/091919 A1.

### - Local treatment of malignant melanoma

As outlined above, the present invention provides a solid microbead having an anti-CD28 monoclonal antibody immobilized thereto for use in the local treatment of malignant melanoma.

Preferably, the malignant melanoma is metastatic melanoma.

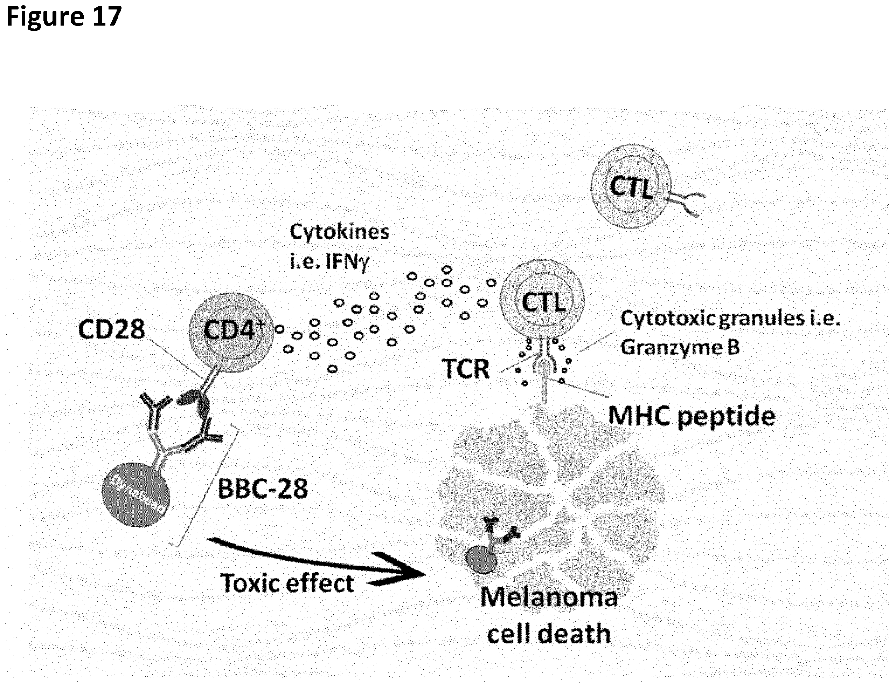

The solid microbeads having an anti-CD28 monoclonal antibody immobilized thereto, as defined herein, are a highly suitable form of potent local immunotherapy. In particular, they deliver a double hit to melanoma metastases (as also shown in Figure 17):
1) enhancing the anti-melanoma activity of T cells (which also leads to the circulation of cancer-reactive and activated T cells to distant metastases and their destruction); and
2) direct cytotoxic effect: the microbeads directly induce cell death in metastatic melanoma and other cancer cells.

In a preferred embodiment, the local treatment comprises intradermal, intralesional and subcutaneous injection.

Preferably, injection into the metastases and the border zone of metastasis and healthy skin. In one embodiment, intralesional administration can comprise image-guided injection into visceral metastases

In one embodiment, the solid microbeads are mixed with autologous T cells for intralesional administration, preferably at a ratio of microbeads to T cell of about 5:1.

The microbeads are preferably suspended in 0.9% sterile saline for injection.

### - Combination with further therapies

In one embodiment, the use is in combination with one or more further therapies for malignant melanoma, preferably for metastatic melanoma.

The one or more further therapies is/are preferably:
- immunotherapy, such as with checkpoint inhibitors,
- targeted therapy, such as with BRAF inhibitors or MEK inhibitors,
- chemotherapy,
- radiotherapy,
- surgical treatment,
or combinations thereof.

### Treatment methods

As outlined above, the present invention provides a method for the local treatment of malignant melanoma.

Said method comprises the step of
administering a therapeutically effective amount of solid microbeads as defined herein or a pharmaceutical composition comprising the solid microbeads as defined herein.

The local treatment preferably comprises intradermal, intralesional and subcutaneous injection.

In a preferred embodiment the administration is intralesional and comprises injection into the macroscopic border area or zone between skin metastases and surrounding healthy skin and directly into skin metastases.

For this administration, the number of microbeads (e.g. BBC-28) injected per metastasis should preferably be between equal and five times the anticipated number of melanoma cells in the metastasis (1 cm³ of melanoma tissue equals about 1 × 10⁸ melanoma cells (DelMonte *et al.* 2009).

The total amount of microbeads (e.g. BBC-28) applied to a metastasis is preferably evenly distributed over different injection sites (≥ 1 cm³ of melanoma tissue: at least ten separate injections). A single injection has preferably a volume of about 10 µl containing 1 to 5 × 10⁷ microbeads (e.g., BBC-28). The microbeads (e.g. BBC-28) are preferably suspended in 0.9% sterile saline for injection. A 27G needle is preferably used for the injections.

In case of visceral metastases (e.g. liver metastases), intralesional therapy/administration can comprise image-guided injection into visceral metastases.

For intralesional application into skin or visceral metastases, the microbeads (e.g. BBC-28) can be mixed with autologous T cells at a microbeads (e.g. BBC-28) to T cell ratio of about 5:1. In case the treatment does not lead to complete destruction of metastatic tissue, microbead application (e.g. BBC-28 application) can be repeated after two to four weeks with an adapted to the anticipated number of remaining melanoma cells.

The malignant melanoma is preferably metastatic melanoma.

A "therapeutically effective amount" of a solid microbead of the present invention refers to the amount which has to be administered to a subject in need thereof in order to achieve a desired therapeutic result or outcome. The skilled artisan will be able to determine said therapeutically effective amount and the suitable administration regimen.

A pharmaceutical composition comprising the solid microbeads as defined herein optionally comprises one or more pharmaceutically acceptable excipients and/or carrier.

Preferably, the pharmaceutical composition is suitable for intradermal, intralesional and subcutaneous injection.

In one embodiment, the method is combined with one or more further therapies for malignant melanoma, preferably for metastatic melanoma.

As discussed above, one or more further therapies is/are preferably:
- immunotherapy, such as with checkpoint inhibitors,
- targeted therapy, such as with BRAF inhibitors or MEK inhibitors,
- chemotherapy,
- radiotherapy,
- surgical treatment,
or combinations thereof.

### Preferred embodiments

### - Abstract

Metastatic disease is the prime cause of death from many malignancies. This is especially true for neoplasms like malignant melanoma or breast cancer where the primary lesions are surgically well accessible often allowing for complete removal. However, even if patients appear to be cured after first line treatment many progress to metastatic disease sometimes years after the first diagnosis was made. Due to the multiple different lesions and the increased aggressiveness of the cancer cells, metastatic disease is notoriously difficult to treat. Here, the introduction of immune checkpoint blockers (ICB) has greatly helped to improve patients' prognosis by harnessing anti-tumour immunity, particularly anti-cancer T cells. Despite the success of ICB in e.g. metastatic melanoma two major challenges remain in the field: Therapy-limiting toxicities induced by systemic ICB administration and lack of a life-prolonging response in the majority of patients. Therefore, highly potent local immunotherapeutic approaches are required to enhance treatment of metastatic disease. We here describe antibody-functionalised paramagnetic microbeads as a means to fill this void. The microbeads are readily engulfed by the vast majority of human cancer cell line cells studied inducing direct cytotoxicity (first hit). Focussing on malignant melanoma, we further observed that loading the microbeads with a conventional anti-CD28 mAb (bead-bound conventional anti-CD28 mAb, BBC-28) not only efficiently induced proliferation and cytokine release by human T cells expressing CD28, but also enhanced killing of melanoma cells by melanoma-reactive T cells (second hit). We, thus, provide proof-of-concept data that BBC-28, after local application into e.g. skin metastases, induces a combination of direct toxicity and enhanced anti-cancer immunity, leading to efficient treatment of metastatic disease.

### - Results

### Microbeads constitute a substrate for immobilization of conventional monoclonal anti-CD28 antibodies

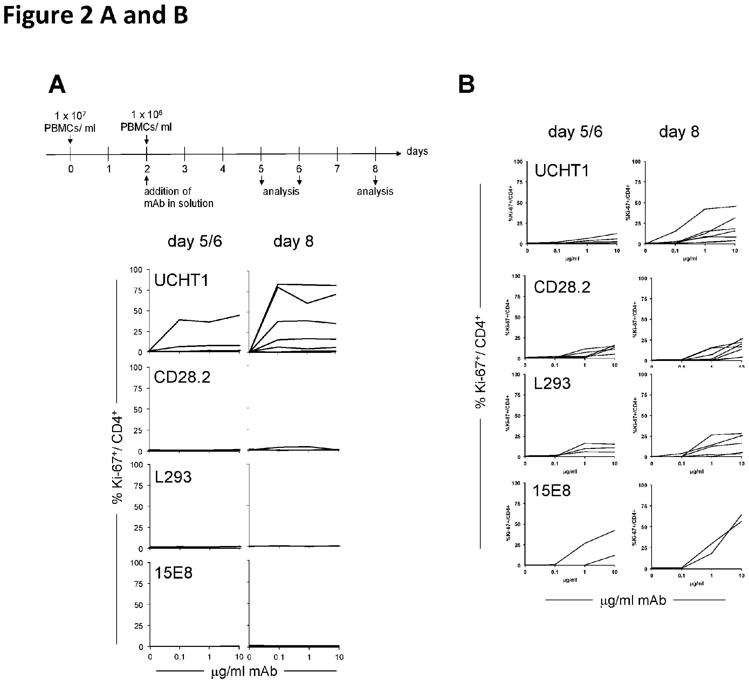

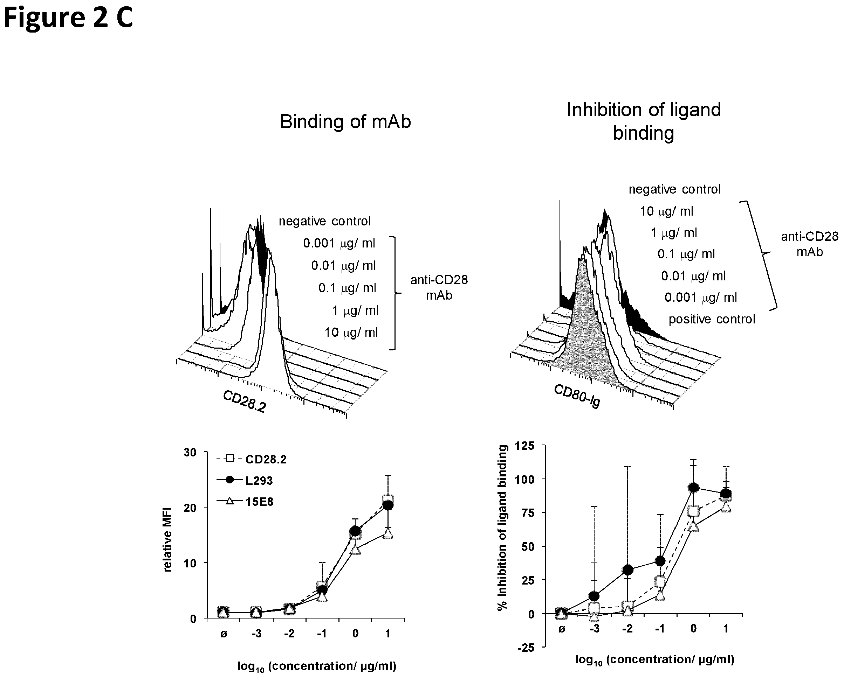

In order to generate a product for strictly local immune stimulation the inventors immobilized a conventional anti-human CD28 mAb (clone 15E8) on Dynabeads^{™} Pan Mouse IgG, as previously described by the inventors in international patent application WO 2015/091919 A1. Conventional anti-CD28 mAb (clones CD28.2, L293 and 15E8) did not induce T cell proliferation in solution, as read out by measuring expression of Ki-67, even after so-called high-density culture after which superagonistic anti-CD28 mAb like TAB08/ TGN1412 by themselves strongly activate T cells (Dennehy *et al.,* 2003; Bischof *et al.,* 2000) (Figure 2A). Immobilized on Dynabeads^{™} Pan Mouse IgG, however, conventional anti-human CD28 mAb, including clone 15E8, are capable of inducing T cell proliferation (bead-bound conventional anti-CD28 mAb, BBC-28, Figure 2B). Moreover, conventional anti-CD28 mAb not only bind to the CD28 receptor on primary human CD4⁺ T cells dose-dependently (Figure 2C, left column), but also block binding of a soluble ligand for CD28, CD80-Ig (R&D Systems), to CD28 expressed by primary human CD4⁺ T cells (Figure 2C, right column). Therefore, conventional anti-human CD28 mAb, including clone 15E8, do not bind the C"D loop of the CD28 molecule to which superagonistic anti-CD28 mAb are known to bind (Beyersdorf, Hanke *et al*., 2005).

### BBC-28 stimulation leads to CD4⁺ and CD8⁺ T cell proliferation in vitro

As BBC-28 (anti-CD28 mAb immobilized on Dynabeads^{™}) was proposed to stimulate anti-tumor memory CD4⁺ T Cells, the proliferation of T cells induced by BBC-28 was investigated prior to the investigation of the anti-tumor activity obtained from T cells stimulated with BBC-28.

In order to determine the stimulatory and mitogenic abilities of BBC-28 on CD4⁺ and CD8⁺ T cells, Dynabeads^{™} Pan Mouse IgG were coated with 10µg/ml anti-CD28 mAb (clones CD28.2 or 15E8) and cocultured with PBMCs for six days. Alternatively, Dynabeads^{™} were coated with normal mouse immunoglobulin (nmlg) (20µg/ml) as a negative control or with a mixture of anti-CD3 and anti-CD28 (clone CD28.2) mAb (0.1µg/ml) as a positive control. As another positive control the commercially available Dynabeads^{™} Human T-Activator CD3/CD28 were included.

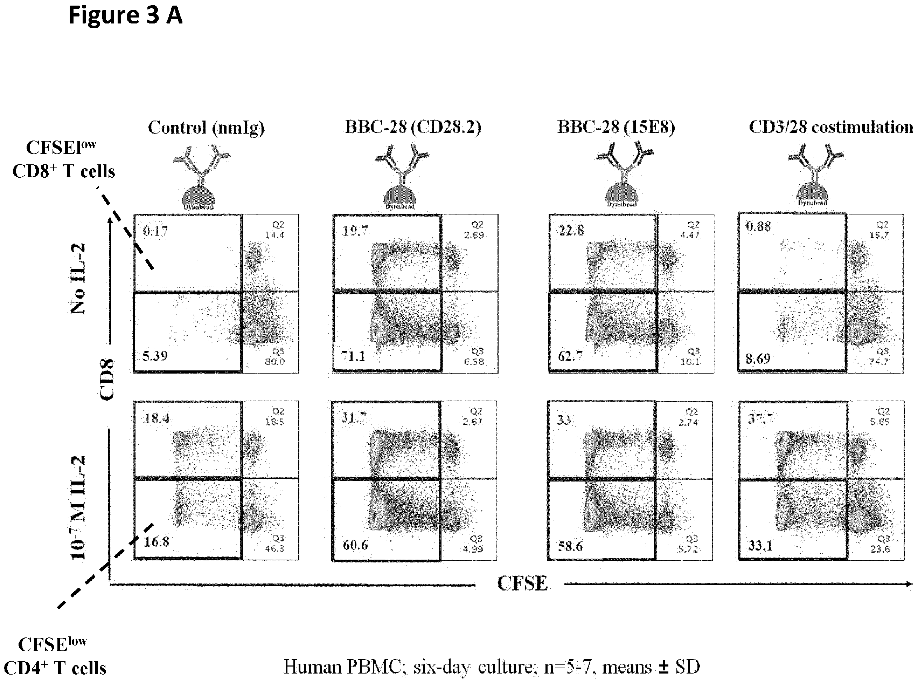

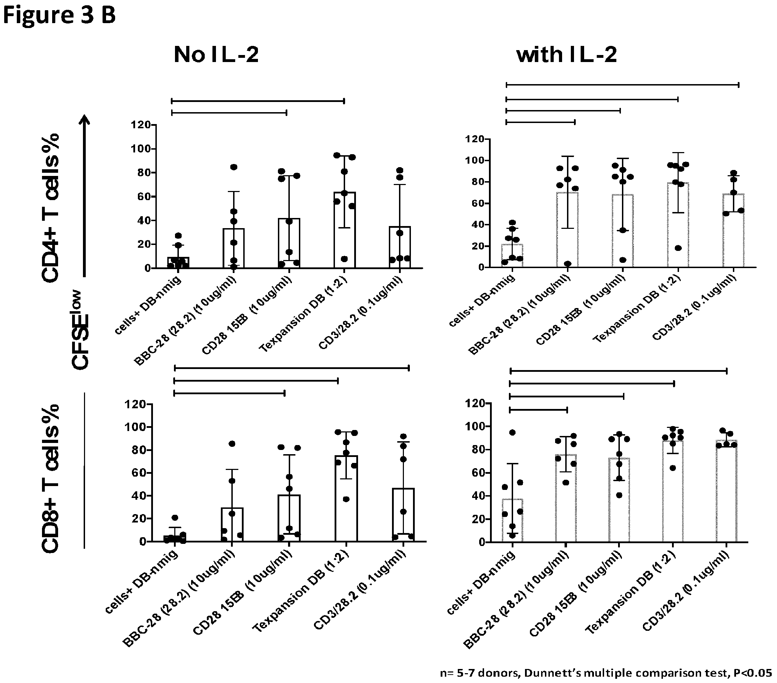

T cell proliferation was evaluated by flow cytometry revealing that CD4⁺ (lower bold boxes in Figure 3A) and CD8⁺ (upper bold boxes in Figure 3A) T cells proliferated upon being stimulated by BBC-28 independent of the anti-CD28 mAb clone immobilized on the Dynabeads^{™} (Figure 3A). In the absence of rhIL-2, BBC-28 (clone 15E8) stimulation of PBMCs resulted in a significant proliferation of CD4⁺ and CD8⁺ T cells (Figure 3B) as the frequency of CFSE^{low} cells among CD4⁺ T cells increased four-fold and of CFSE^{low} cells in CD8⁺ T cells increased even eight-fold compared to control. This indicates the stronger ability of clone 15E8 to induce proliferation of CD4⁺ and CD8⁺ T cells compared to clone CD28.2. The proliferation was generally enhanced in the presence of rhIL-2. BBC-28-induced proliferation of CD4⁺ and CD8⁺ T cells was found to be very similar to that induced by the same number of the anti-CD3/ anti-CD28 mAb-Dynabeads^{™} coated in-house irrespective of whether rhIL-2 had been added to the cultures or not (Figure 3B). This indicates that BBC-28 prepared using clone 15E8 were of similar potency regarding induction of T cell proliferation as the widely used anti-CD3/ anti-CD28 mAb-coated Dynabeads^{™}.

### BBC-28 stimulation of PBMCs induces Granzyme B expression in CD8⁺ T Cells

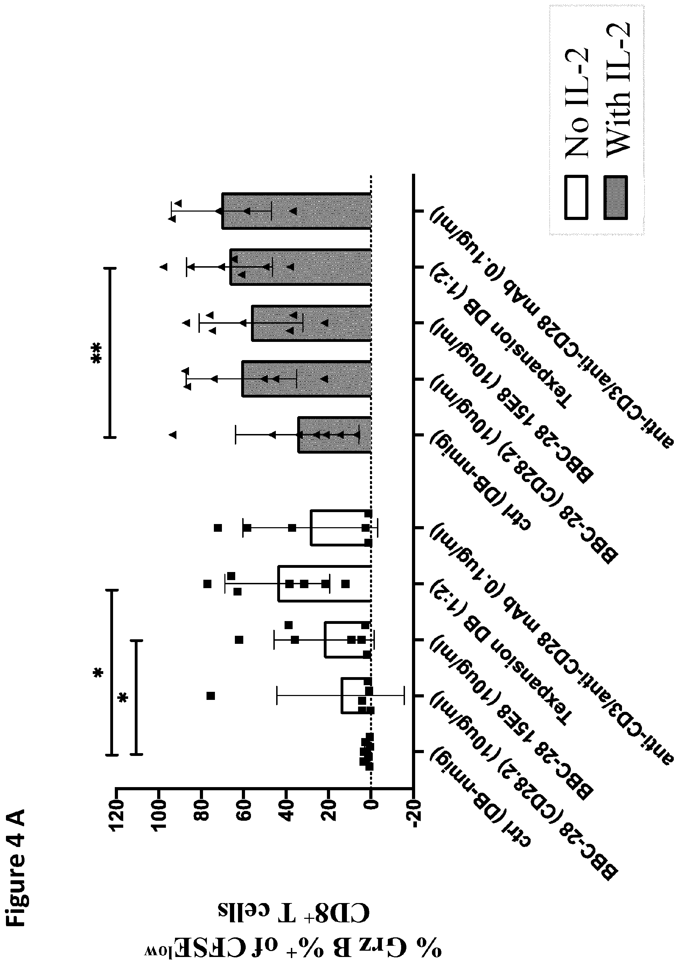

To assess the impact of BBC-28 stimulation on the cytotoxic activity of CD8⁺ T cells, the inventors analyzed expression of Granzyme B by flow cytometry. The flow cytometric analysis revealed that proliferating (CFSE^{low}) CD8⁺ T cells upregulated Granzyme B expression after stimulation with BBC-28 (Figure 4A). BBC-28 prepared with clone 15E8 increased Granzyme B expression by CD8⁺ T cells significantly in the absence of rhIL-2 relative to control (white columns). This significant increase was similar to the increase induced by the in-house coated anti-CD3/ anti-CD28 mAb Dynabeads^{™} which we used as a positive control (white columns) (Figure 4A). Upregulation of Granzyme B expression was generally promoted in the presence of rhIL-2. Thus, BBC-28 stimulation of PBMCs can induce Granzyme B expression by CD8⁺ T cells and clone15E8 was more potent in inducing Granzyme B expression than clone CD28.2.

### BBC-28 stimulation induces IFNy and IL-5 secretion by human PBMC

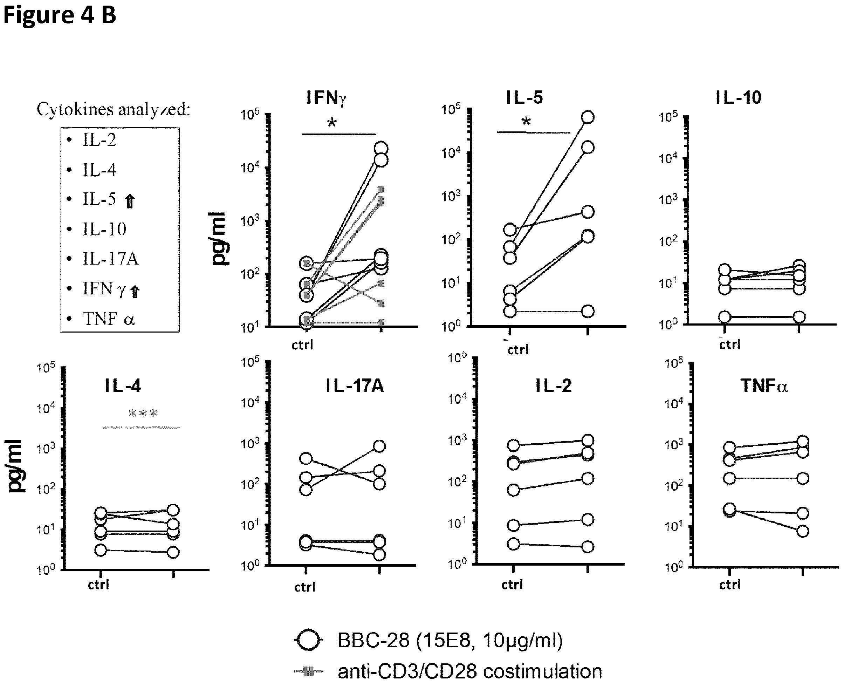

As cytokines play a pivotal role in anti-cancer immunity cytokine concentrations in culture supernatants of PBMCs stimulated with BBC-28 were measured by Legendplex assay. Only IFNγ and IL-5 secretion were significantly increased by BBC-28 stimulation for three days in the absence of rhIL-2 compared to control (Figure 4B). The induction of IFNγ secretion by BBC-28 stimulation was even higher than that induced by the commercially available Dynabeads^{™} Human T-Activator CD3/CD28 which was also not statistically significant. Additionally, BBC-28 did not increase IL-4 secretion in contrast to the Dynabeads^{™} Human T-Activator CD3/CD28 which significantly induced IL-4 secretion. There was no change in the secretion of any of the other cytokines measured upon BBC-28 or Dynabead^{™} Human T-Activator CD3/CD28 stimulation of PBMC.

### BBC-28 stimulation of melanoma cell line-primed T cells enhances killing of melanoma cells

To directly evaluate the suitability of BBC-28 as a novel form of anti-melanoma immunotherapy, the cytotoxic effect of T cells stimulated with BBC-28 was assessed in 2D killing assays. For this purpose, melanoma cell line specificity was first generated in an allogeneic mixed leukocyte reaction (allo-MLR) of isolated human T cells (Pan T cell isolation kit, Miltenyi Biotec) cocultured for six days with Mitomycin C-treated melanoma cells plus 10⁻⁷ M recombinant human Interleukin-2 (Proleuki^{Ⓒ}) (Figure 6). In the ensuing cytotoxicity assay (Figures 7 and 8), melanoma-primed T cells in the presence of BBC-28 (open circles), but not DB-nmIg control (gray squares), showed, after four hours, enhanced killing of SK-MEL-28 cells compared to melanoma-primed T cells only (black triangles) (Figure 9). After 24 h of co-culture, killing of SK-MEL-28 cells was enhanced in the presence of DB-nmIg controls as well as BBC-28 compared to the killing observed with primed T cells only. Thus, BBC-28 were found to effectively enhance killing of SK-MEL-28 melanoma cells by primed T cells.

To investigate priming and efficacious of BBC-28 against other melanoma cell line cells, an analogous experimental set-up was followed for the MeWo melanoma cell line.

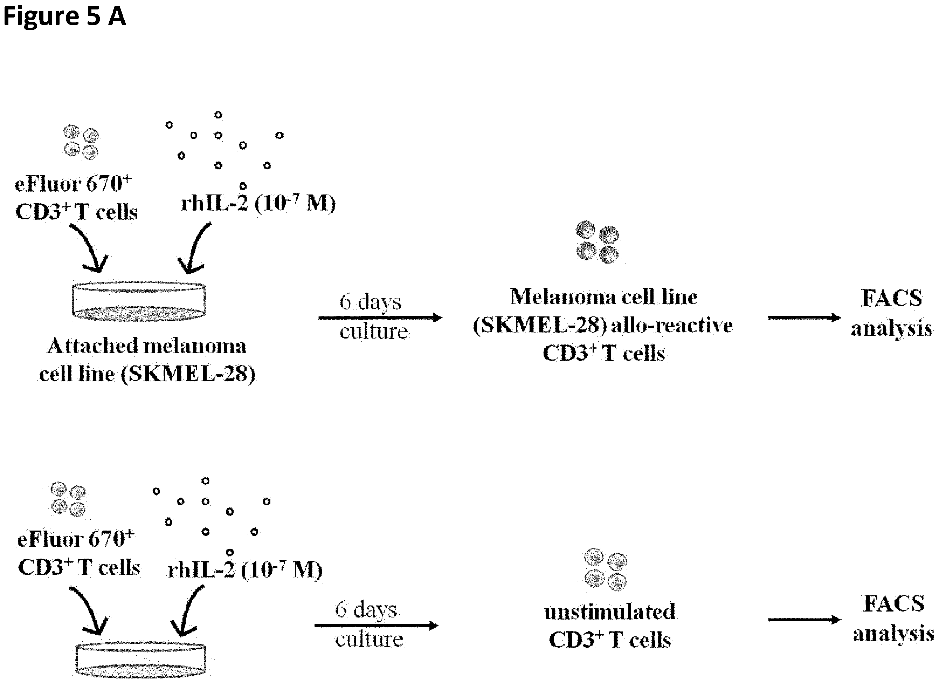

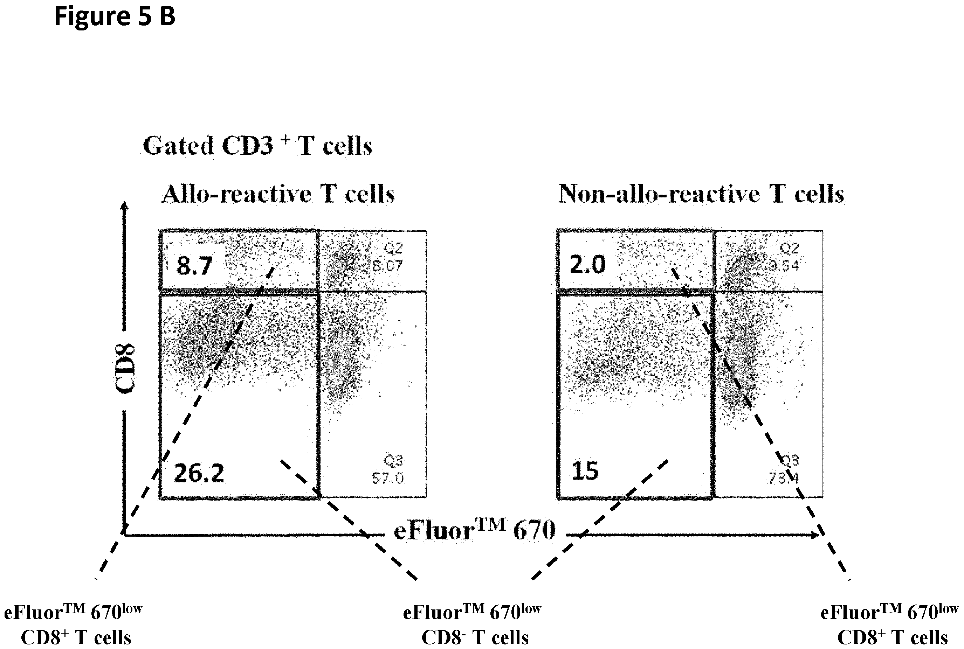

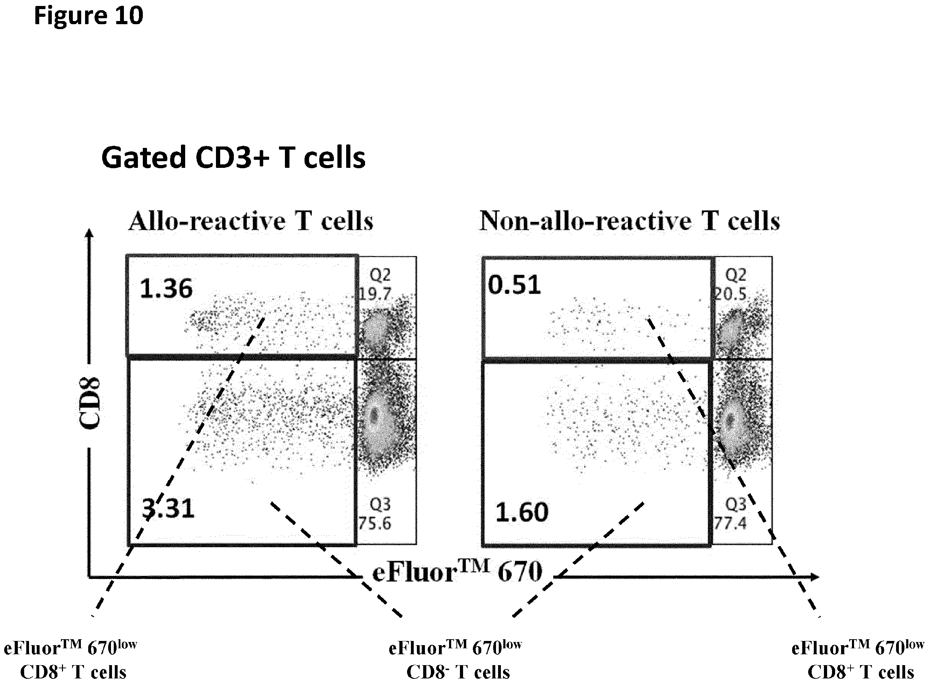

Allo-MLR cultures using eFluor^{™} 670-labeled CD3⁺ T cells were run to compare the degree of allo-reactivity of CD8⁺ and CD8⁻(CD4⁺) CD3⁺ T cells (Figure 5A) towards SK-MEL-28 and MeWo cells. eFluor^{™} 670^{low} dilution was measured by flow cytometry reflecting the percentage of proliferating cells in response to allogeneic MHC molecules expressed by SK-MEL-28 and MeWo cells, respectively (Figure 5B and 10).

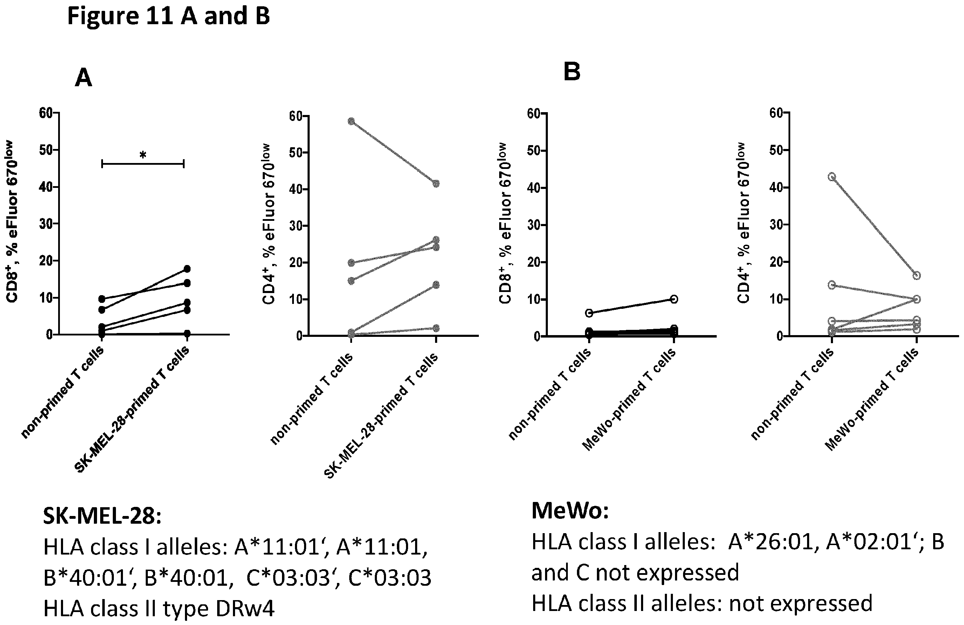

Individual percentages of eFluor^{™} 670^{low} cells among primed CD8⁺ and CD8⁻(CD4⁺) T cells were compared to the percentages of eFluor^{™} 670^{low} cells among non-primed CD8⁺ and CD4⁺ T cells for both SK-MEL-28 and MeWo-primed T cells. SK-MEL-28 cells were more effective in priming T cells (Figure 11A) than MeWo cells (Figure 11B) and only SK-MEL-28 cell-primed T cells showed a significant, 3.57-fold, increase in the proportion of eFluor^{™} 670^{low} cells among CD8⁺ T cells (around 5% of CD8⁺ T cells responded to allogenic MHC class I molecules) compared to non-primed CD8⁺ T cells.

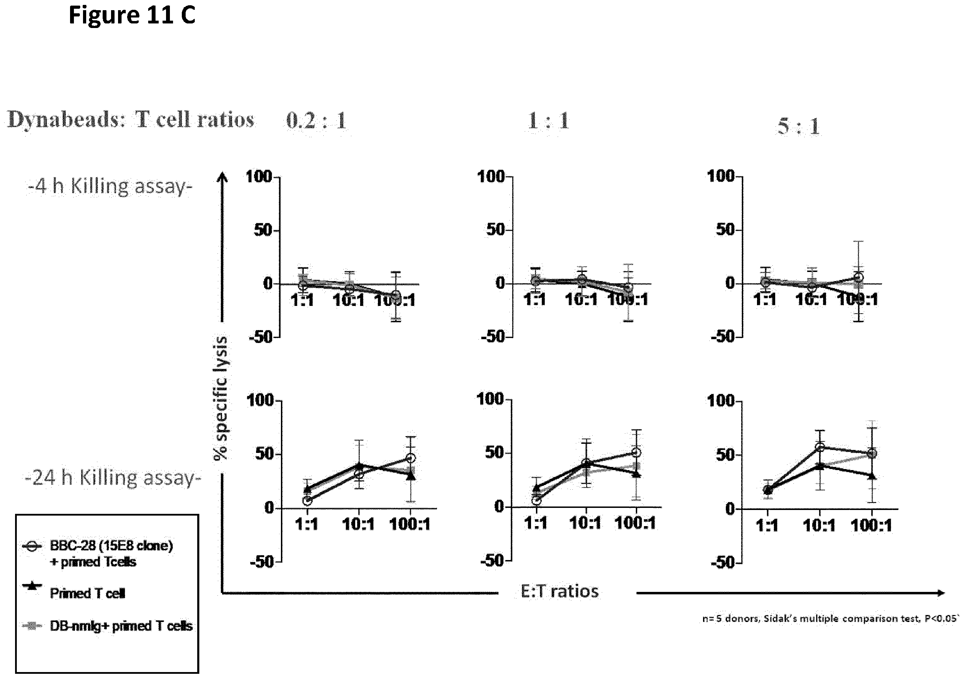

Regarding cytotoxic activity, BBC-28 were found to be inefficient in inducing MeWo cell killing after 4 h of co-incubation (Figure 11C). After 24 h, the overall killing of MeWo cells by primed T cells was increased compared to 4 h of incubation. Yet, BBC-28 failed to further enhance the cytotoxic activity of the primed T cells even after 24 h.

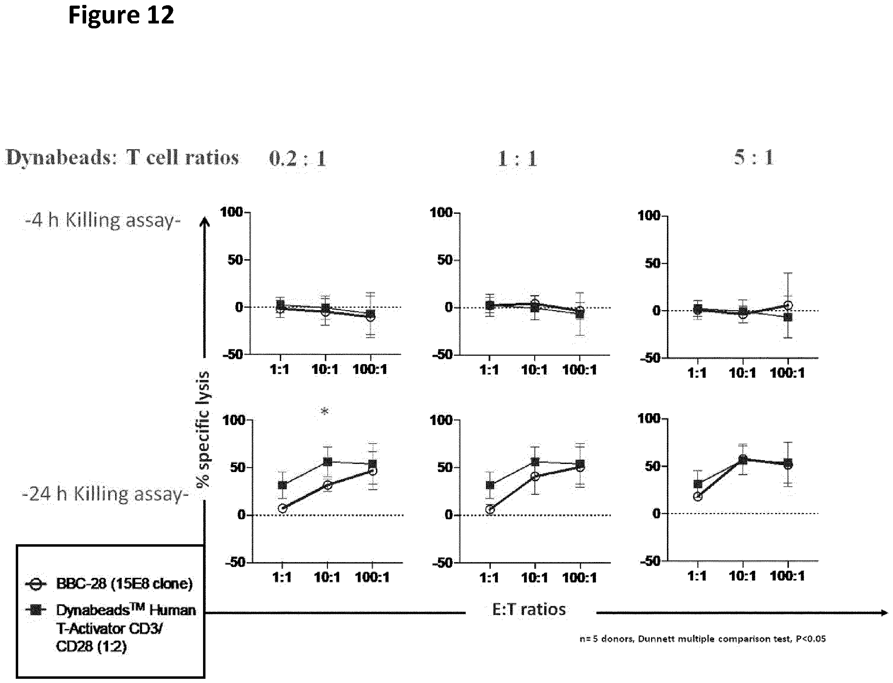

To test whether the degree of stimulation induced by BBC-28 was not sufficient to enhance the killing activity of primed T cells against the MeWo cell line, the inventors added the commercially available Dynabeads^{™} Human T-Activator CD3/CD28 in parallel cultures (Figure 12). However, also there the inventors observed only weak specific lysis of the MeWo cells dependent on the E: T and the DB: T cell ratios and the coincubation period. Therefore, poor priming of T cells against MeWo melanoma cell line cells correlated with a failure to respond to T cell-activating microbeads in the killing assay.

### BBC-28 was better than ICB in enhancing killing of melanoma cells by primed T cells in 2D cytotoxicity assays

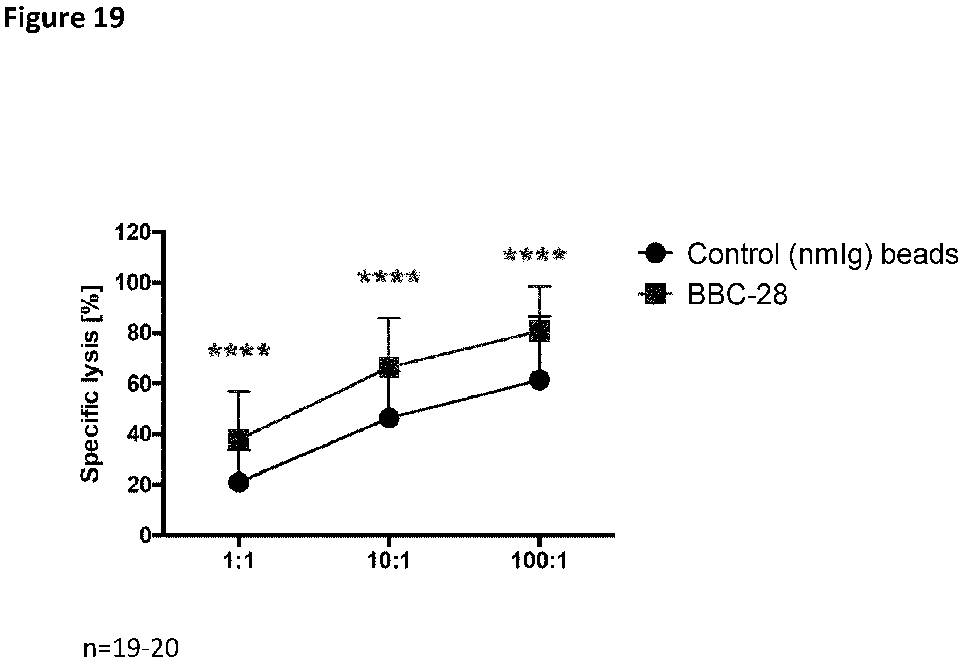

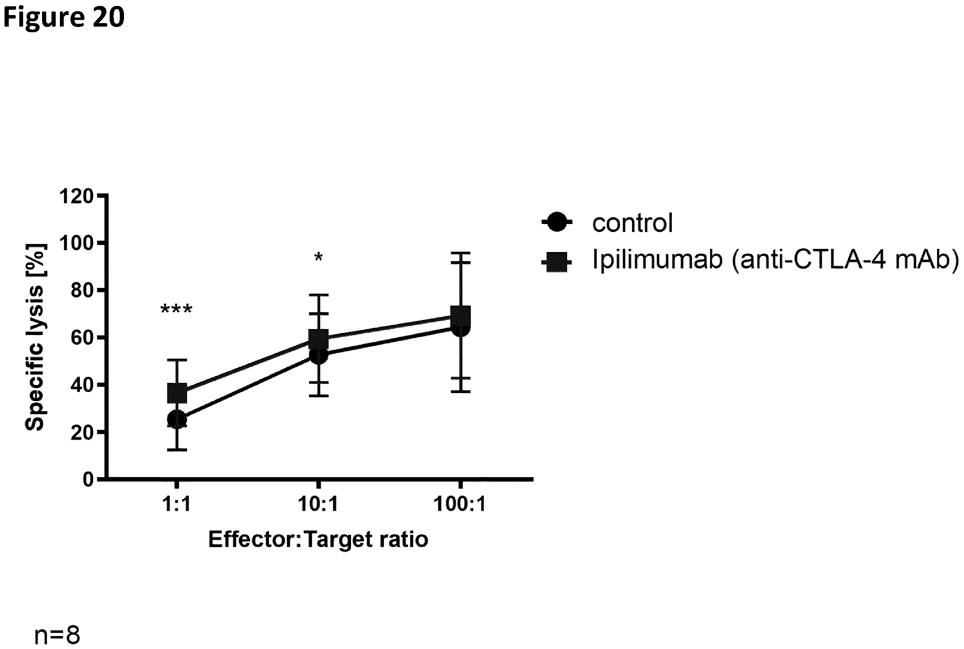

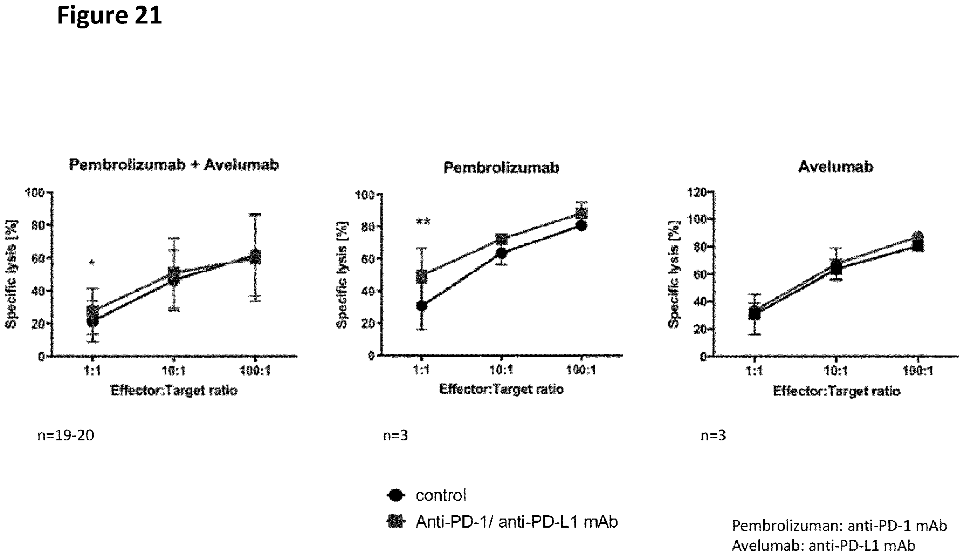

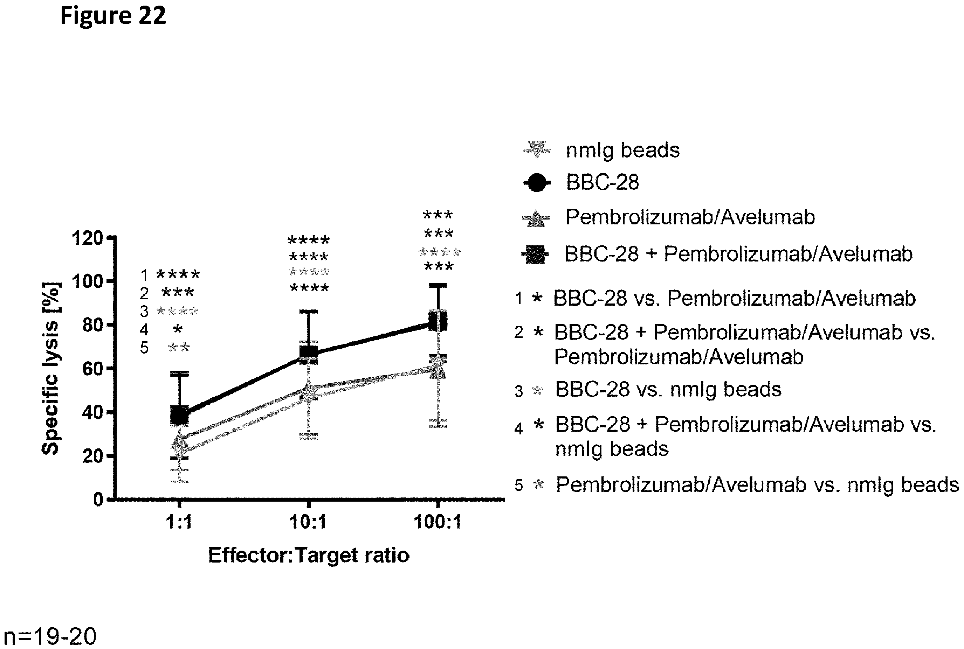

To determine how BBC-28-mediated induction of melanoma cell killing would compare to ICB-induced killing we conducted another set of experiments using SK-MEL-28-primed nylon wool-non adherent T cells. Similar to T cells isolated by negative magnetic isolation (Figure 12), BBC-28 also enhanced killing of SK-MEL-28 cells by nylon wool-non adherent T cells (Figure 19). Blocking CTLA-4 with Ipilimumab (Figure 20) as well as PD-1 and/or PD-L1 with Pembrolizumab and/ or Avelumab also enhanced melanoma cell killing by primed T cells with the exception of cultures in the presence of Avelumab only (Figure 21). Combining BBC-28 with Pembrolizumab and Avelumab did not enhance killing by primed T cells compared to addition of BBC-28 only (Figure 22). Therefore, the priming protocol and ensuing 2D cytotoxicity assay used by the inventors were suitable to detect a beneficial effect of ICB on melanoma cell killing by T cells. Moreover, BBC-28 increased killing of melanoma cells more profoundly than ICB and there was no additional or synergistic effect by combining ICB and BBC-28 versus ICB or BBC-28 alone.

### Dynabeads^{™} exert a direct toxic effect on melanoma and other cancer cell line cells

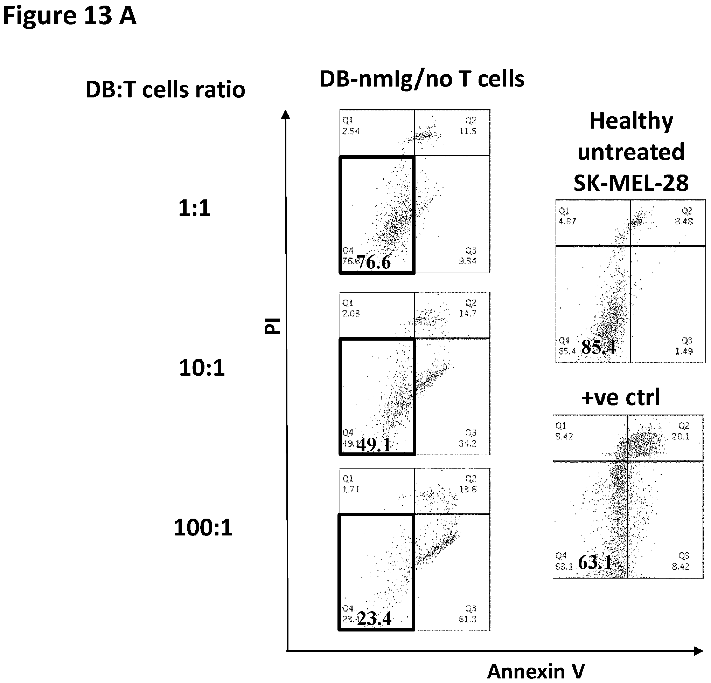

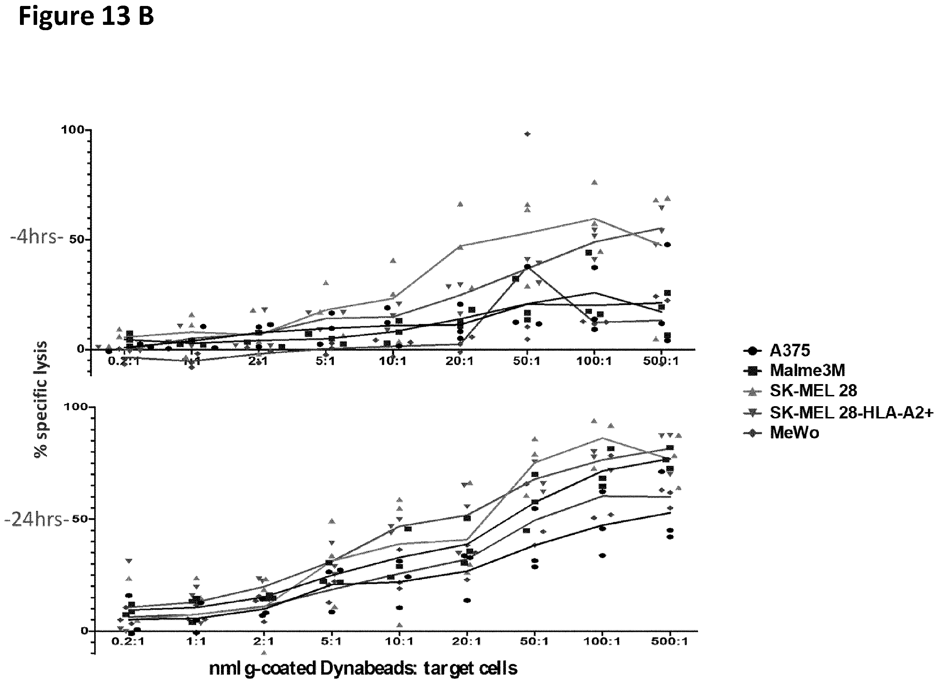

Unexpectedly, the inventors observed the strongest killing of SK-MEL-28 cells in control cultures without T cells (Figure 13A). % specific killing positively correlated with the DB: melanoma cell ratio indicating that Dynabeads^{™} are per se toxic to SK-MEL-28 cells. To investigate whether Dynabeads^{™} are also toxic to other melanoma cell line cells, cells from five different melanoma cell lines (namely A375, Malme3M, SK-MEL-28, SK-MEL-28-HLA-A2⁺ and MeWo) were co-incubated with DB-nmIg for 4 and 24 h. Dynabeads^{™} were found to be toxic for the five melanoma cell line cells tested (Figure 13B). The cell lines, however, showed different degrees of susceptibility to Dynabead^{™}-mediated toxicity (Figure 13B). Dynabeads^{™} induced toxicity was enhanced by the prolongation of the coincubation period from four to 24 h.

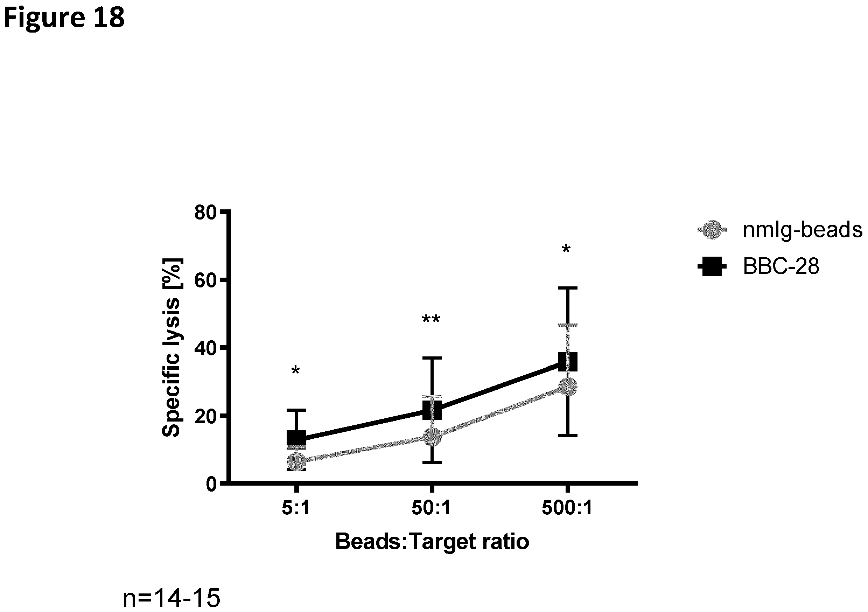

Regarding the coating of the Dynabeads^{™} Pan Mouse Ig, we observed that BBC-28 were slightly more toxic to SK-MEL-28 cells than DB-nmIg (Figure 18).

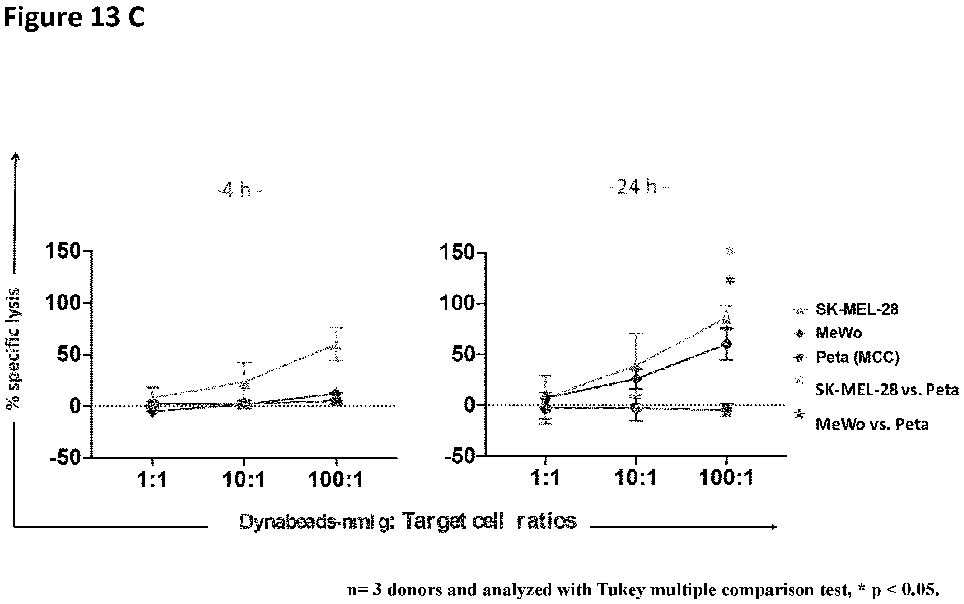

To investigate whether Dynabeads^{™} can also be toxic to other skin cancer types, DB-nmIg were co-incubated with the merkel cell carcinoma cell line, PeTa (Schrama *et al.,* 2019). PeTa cells were found to be resistant to Dynabead^{™}-induced toxicity even after 24 h of coincubation in contrast to the sensitive SK-MEL-28 cells and the moderately sensitive MeWo cells (Figure 13C).

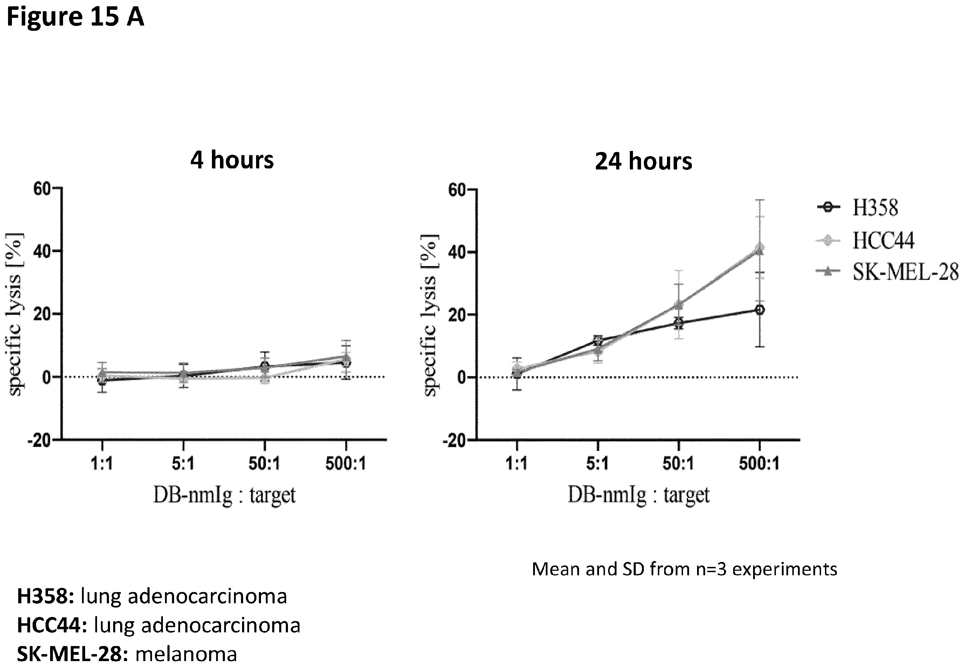

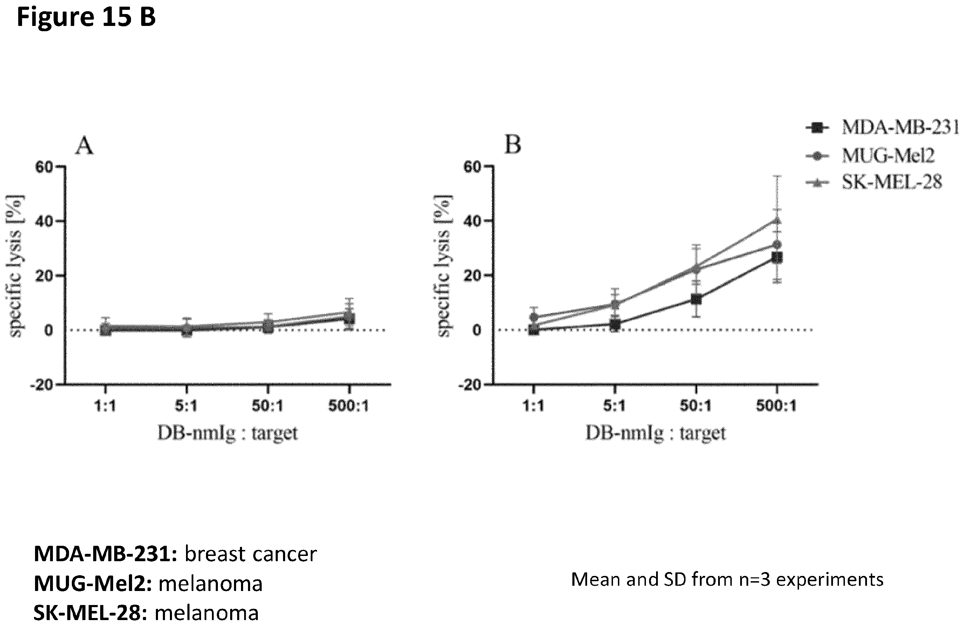

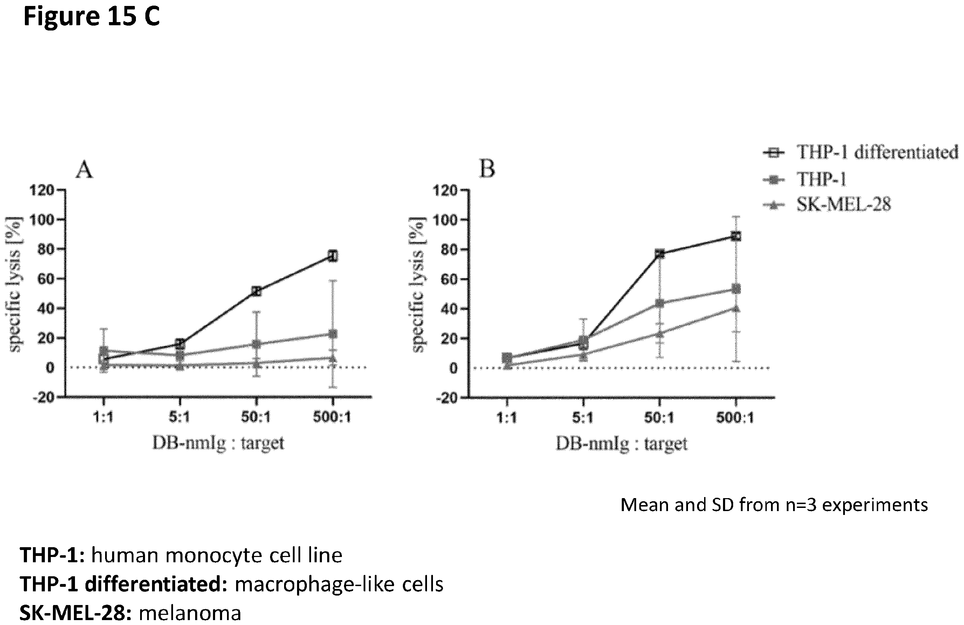

To further broaden the spectrum of cancer entities analyzed, the toxicity of Dynabeads^{™} was investigated against THP-1 (monocytic leukemia), differentiated macrophages from THP-1, MDA-MB-231(breast adenocarcinoma), HCC44 and H358 (lung adenocarcinoma) and MUG-Mel 2 (melanoma) cell line cells and compared to the toxicity against SK-MEL-28 cells (Figure 15). All tested cell lines from these different cancer types were found similarly sensitive to Dynabead^{™}-induced toxicity as SK-MEL-28 cells with the exception of THP-1 and differentiated macrophages from THP-1 that showed superior sensitivity to Dynabead^{™}-mediated toxicity.

### Internalization of Dynabeads^{™} induces direct cytotoxicity in cancer cell line cells

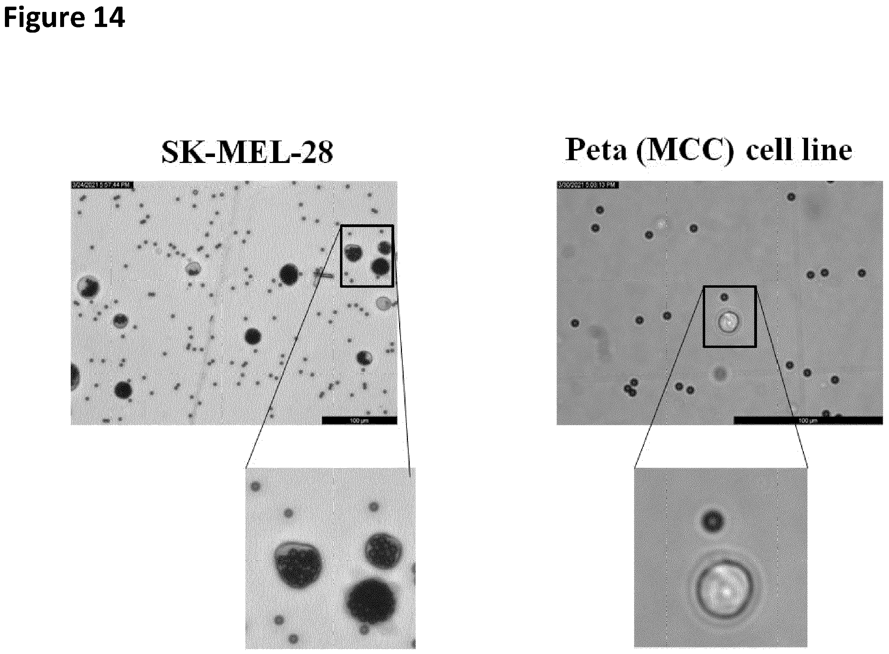

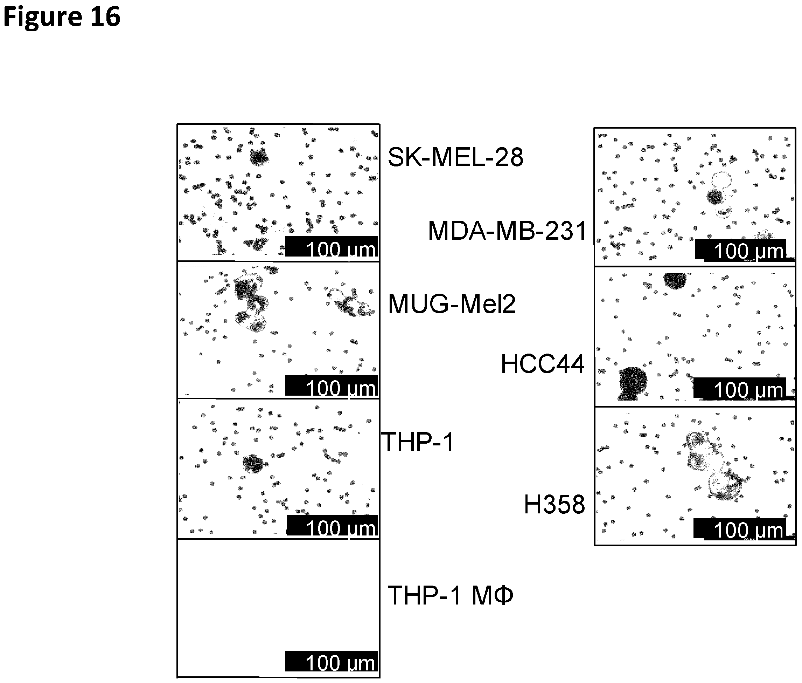

To investigate the possible reason for Dynabead^{™}-induced toxicity, cell line cells coincubated with DB-nmIg were analyzed by light microscopy. Microscopy revealed that all sensitive cell lines were able to internalize and accumulate the Dynabeads^{™} inside (Figure 16) in contrast to the resistant PeTa cell line cells that did not (Figure 14).

Thus, the data described herein show that BBC-28 is suitable as an effective anti-melanoma immunotherapy by enhancing anti-melanoma T cell activity and by exerting a direct cytotoxic effect on the melanoma cells due to internalization of the microbeads.

### Discussion

Physiologically, CD28 costimulation requires CD28 ligands (B7 molecules) on antigen-presenting cells (APCs) to bind to the CD28 receptor initiating T cell stimulation (see e.g. Tan *et al.,* 1993). Conventional anti-CD28 mAb by themselves are not able to activate T cells (reviewed in Poirier *et al.,* 2012). Full T cell activation requires the presence of a stimulus for the TCR complex (Ag or anti-CD3 mAb) plus CD28 costimulation (see e.g. Sanchez-Lockhart *et al.,* 2014). The inventors had found, see international patent application WO 2015/091919 A1 or European patent application 2 886 645 A1, that by immobilizing conventional anti-CD28 mAb on a backbone matrix acting like an artificial APC, i.e. Dynabeads^{™}, these anti-CD28 mAb-coated artificial APCs (BBC-28) were able to activate the CD28 receptor and induce cellular responses like e.g. proliferation.

As disclosed herein, the inventors found that BBC-28 were able to stimulate CD4⁺ and CD8⁺ T cells among PBMC (as shown in Figure 3). Especially clone 15E8 induced significant proliferation of T cells (as shown in Figure 3). Moreover, BBC-28 stimulation of T cells was found to be similarly potent in inducing T cell proliferation as anit-CD3/anti-CD28 mAb-mediated costimulation. The addition of rhIL-2 to the culture enhanced proliferation in general, irrespective of whether CD3 and CD28 or CD28 alone had been stimulated.

BBC-28 stimulation of PBMCs upregulated the expression of Granzyme B by CD8⁺ T cells that was again significant for clone 15E8 (as shown in Figure 4A). Additionally, BBC-28 stimulation of PMBC significantly induced IFNγ secretion, while IFNγ secretion induced by anti-CD3/anti-CD28 mAb-stimulated PBMC did not reach statistical significance in our experimental series (see Figure 4B). The strong release of IFNγ upon BBC-28 stimulation of PBMC indicates Tₕ1 cell activity (Curtsinger and Mescher, 2010). In parallel, BBC-28 stimulation also induced secretion of the Tₕ2 cytokine IL-5. IL-4, another key Tₕ2 cytokine was, however, only induced by anit-CD3/anti-CD28 mAb-mediated costimulation. We assume that the induction of Granzyme B expression as well as IFNγ and IL-5 secretion by BBC-28 supports the cytotoxic activity of the stimulated T cells (reviewed in Boivin *et al*., 2009).

To test the cytotoxic activity of T cells stimulated by BBC-28, the inventors first primed T cells against melanoma cells in allo-MLR cultures. The percentage of allogeneic T cells responding depended on the MHC molecules expressed: SK-MEL-28 cells, which express six MHC class I and two MHC class II molecules, primed T cells better (as shown in Figure 11A) than MeWo cells which express only two MHC class I molecules and no MHC class II molecules (see Figure 11B) (Boegel *et al.,* 2014).

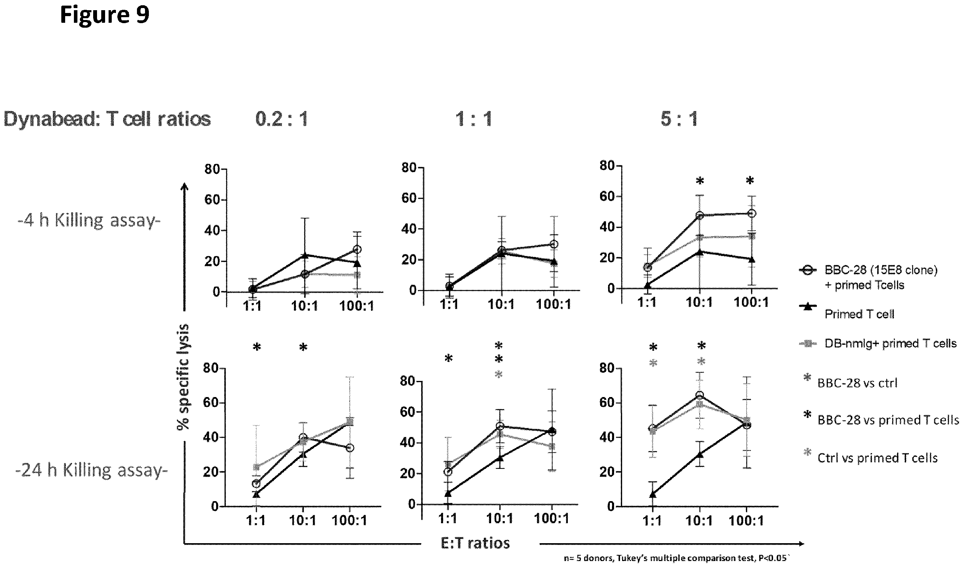

The specific killing of the allo-reactive T cells stimulated with BBC-28 was then evaluated in killing assays against SK-MEL-28 and MeWo cells. The stimulation of primed T cells by BBC-28 enhanced the killing of melanoma cells better than control Dynabeads^{™} (DB-nmIg). Furthermore, SK-MEL-28 cells (see Figure 9) that primed T cells better than MeWo cells, showed higher specific lysis relatively to MeWo cells (shown in Figure 11C).

In patients, ICB are now widely used for the treatment of metastatic melanoma. However, showing beneficial effects of ICB *in vitro* remains notoriously difficult (Kim *et al.,* 2022). Therefore, it is important to note that the protocol used by us to prime T cells against melanoma and the 2D cytotoxicity assay were suitable to detect an increase in melanoma cell killing in the presence of ICB. This indicates that the positive results obtained with BBC-28 in the inventors' priming and cytotoxicity assay are of predictive value regarding future applications in patients.

In addition to T cell-mediated killing, the inventors found a direct toxic effect of the Dynabdeads^{™} on the melanoma cells. Here, it is important to note that this effect was in principle independent of whether the Dynabdeads^{™} Pan Mouse IgG had been coated with nmlg or anti-CD28 mAb (see Figure 18).

The toxicity of Dynabdeads^{™} was further investigated against other melanoma, monocytic leukemia, breast and lung adenocarcinoma cell line cells (Figures 13B and 15). All tested cell lines were found to be susceptible to Dynabead^{™}-mediated toxicity. This was in contrast to the PeTa cell line, a merkel cell carcinoma (Schrama *et al.,* 2019), that was found to be resistant to Dynabead^{™}-induced toxicity (shown in Figure 13C). The inventors thus found that Dynabead^{™}-induced toxicity was positively correlated with the capacity of the cancer cell line cells to internalize and accumulate the Dynabeads^{™} inside (see Figures 14 and 16) suggesting that microbead internalization was the key step to induction of cell death.

Regarding the mechanism by which cancer cells incorporate the microbeads, the inventors assume that this occurs due to phagocytosis as phagocytosis is a cellular process to ingest particles larger than 0.5 µm in diameter, which applies to the Dynabeads^{™} used in our study having diameter of 4.5µm. Additionally, phagocytosis can also be accomplished by non-professional phagocytes such as fibroblasts, endothelial, epithelial and most notably also melanoma cells (see e.g. Uribe-Querol *et al.,* 2020). In melanoma, the components of the phagocytic machinery have been shown to be expressed in metastatic disease and to positively correlate with aggressiveness (Lugini *et al.,* 2003). Therefore, the inventors' data are fully in line with published work regarding the capacity of metastatic melanoma cells to phagocytose microparticles.

With the THP-1 cell line the inventors not only analyzed the susceptibility of monocytic leukemia cell line cells towards Dynabead^{™}-induced toxicity, but also differentiated them into macrophage-like cells. These macrophage-like THP-1 cells were very efficiently killed when co-cultured with Dynabeads^{™} (as shown in Figure 15). This is an important observation regarding the application of BBC-28 to melanoma lesions in patients: BBC-28 can beneficially modulate the tumor microenvironment by killing/ reprogramming tumor-associated macrohphages- key mediators of cancer immune evasion- and thus function as an adjuvant for the T cell activation induced by BBC-28. Moreover, induction of cell death induced in monocyte-like THP-1 cells suggest that also monocytes in patients *in vivo* can die after phagocytosing the microbeads thereby reducing the risk that monocytes might transport the microbeads to remote parts of the body where they might cause unwanted side effects like microembolism.

For the treatment of cancer, a number of nanoparticles, mostly liposomes, are approved for clinical use (reviewed in Kamata and Tada, 2021). Moreover, many different forms of nanoparticles with a diameter of 20 to 200 nm are currently under preclinical and early clinical development (La-Beck *et al.,* 2020). In contrast to nanoparticles, far fewer microparticle preparations are already in clinical use in humans. A key application for microparticles is transcatheter vessel occlusion for which microparticles with diameters of mostly more than 100 µm are used (Sheth *et al.,* 2017). Experimentally, it has been shown that Dynabeads^{™} with a diameter of 4.5 µm, such as the ones used in the present invention, could be repeatedly administered i.v. into mice as so-called artificial antigen-presenting cells (APC) to enhance anti-tumour immune responses (Ugel *et al.,* 2009). However, it has also been shown that Dynabeads^{™} with a diameter of 4.5 µm end up in capillaries and, thus, pose a risk for inducing microembolisms (Takemoto *et al.,* 2002). Local application of BBC-28 to metastatic lesions can avoid such complications.

In summary, the results disclosed herein show that BBC-28 is highly suitable as a novel form of potent local immunotherapy. BBC-28 are capable of delivering a double hit to melanoma metastases (as shown in Figure 17) by enhancing the anti-melanoma activity of T cells which ideally also leads to the circulation of cancer-reactive and activated T cells to distant metastases and their destruction. BBC-28 can, thus, function as a trojan horse. The second hit stems from the capacity of microbeads, i.e. the Dynabeads^{™} with a diameter of 4.5 µm used in our study, to directly induce cell death in metastatic melanoma and other cancer cells.

The following examples and drawings illustrate the present invention without, however, limiting the same thereto.

### BRIEF DESCRIPTION OF THE DRAWINGS

**Figure 1****.** *Solid microbeads coated with an anti-mouse Ig monoclonal antibody to which an anti-CD28 monoclonal antibody is bound (also called "BCC-28*"*) as used according to the invention.*
**Figure 2****.** *Mitogenic capacity of microbead-immobilized conventional anti-CD28 mAb.*
   **A)** Stimulation of PBMCs after high-density pre-culture in the presence of titrated amounts of soluble or **B)** Dynabeads^{™} Pan Mouse IgG-bound anti-CD3 (clone UCHT) or anti-CD28 mAb (clones CD28.2, L293 and 15E8). The percentage of proliferating, i.e. Ki-67⁺ cells, was determined after five to six (left column) and eight days of culture (right column), respectively. Each line reflects the response of CD4⁺ T cells from one blood donor.
   **C)** Anti-CD28 mAbs CD28.2, L293 and 15E8 block ligand binding to CD28. The left histogram shows dose-dependent binding of mAb CD28.2 to human CD4⁺ T cells gated among PBMCs. In the summary graph relative median fluorescence intensities (rel. MFI) were determined by dividing the MFI of specifically stained cells by that of the negative control. Means + SD of eleven (CD28.2), six (L293) and three (15E8) individual determinations are depicted. Pre-bound anti-CD28 mAb block the binding of human CD80-Ig to CD28 (right). Means + SD of nine (CD28.2), four (L293) and three (15E8) individual determinations are shown.
**Figure 3****.** *BBC-28 induces CD4*⁺ *and CD8+ T cell proliferation in vitro.*
   **A)** Representative dot plots after six days of co-culture of mAb-coated Dynabeads^{™} with PBMCs in the presence or absence of rhIL-2. T cell proliferation was evaluated using CFSE dilution as readout. Generally, proliferation was enhanced in the presence of rhIL-2.
   **B)** Summary of the frequencies of CFSE^{low} cells among either CD4⁺ or CD8⁺ T cells cultured with (bottom panel) or without (top panel) rhIL-2 for six days in the presence of mAb-coated Dynabead^{™}. Dynabeads^{™} were coated with anti-CD28 mAb 15E8 or CD28.2 (BBC-28), nmlg as negative control (DB-nmIg) or a mixture of anti-CD3 (clone HIT3a)/ antiCD28 mAb as a positive control. As another positive control the commercially available Dynabeads^{™} Human T-Activator CD3/CD28 were included. Results from five to seven independently analyzed donors were pooled and means ± SD are shown. For statistical testing a Dunnett's multiple comparison test was used. ^{∗} p < 0.05, ^{∗∗} p < 0.01, ^{∗∗∗} p < 0.001, ^{∗∗∗∗} p < 0.0001.
**Figure 4****.** *BBC-28 stimulation up-regulated Granzyme B expression by CD8*⁺ *T cells and induced IFNγ and IL-5 secretion by human PBMCs.*
   **A)** Summary of the frequencies of Granzyme B⁺ cells among CFSE^{low} CD8⁺ T cells in the presence or absence of rhIL-2 after stimulation of PBMCs with BBC-28 for six days. BBC-28 are similarly potent in inducing Granzyme B expression in proliferating CD8⁺ T cells as in-house anti-CD3/anti-CD28 mAb-coated Dynabeads^{™} Pan Mouse IgG and the commercially available Dynabeads^{™} Human T-Activator CD3/CD28. Granzyme B expression was increased in the presence of rhIL-2. Results are presented as means ± SD for n = 5-7 donors and were analyzed by Sidak's multiple comparison test. ^{∗} p < 0.05, ^{∗∗} p < 0.01.
   **B)** Cytokine concentrations of IFNγ, IL-5, IL-10, IL-4, IL-17A, IL-2 and TNFα were determined by Legendplex assay in supernatants of BBC-28- or Dynabead^{™} Human T-Activator CD3/CD28-stimulated PBMCs after three days of co-culture in the absence of rhIL-2. Individual results of stimulated cultures are shown in comparison to control (nmlg-coated Dynabeads^{™} Pan Mouse IgG). Results were pooled from n= 6 donors and analyzed with a paired t- test, ^{∗} p < 0.05, ^{∗∗∗} p < 0.001.
**Figure 5****.** *Generation of allo-reactive CTLs to study specific killing of melanoma cells.*
   **A)** Experimental set-up. SK-MEL-28 melanoma cell line-reactive T cells were activated and expanded in an allogeneic mixed lymphocyte reaction. For this, SK-MEL-28 cells were first cultured over night to allow for cell attachment followed by Mitomycin C (10 µg/ml) treatment on the second day. Then purified (negative magnetic isolation) and eFluor^{™} 670-labeled CD3⁺ T cells and rhIL-2 were added and incubated for 6 days (top panel). The degree of SK-MEL-28- (allo-) reactivity of the cultured T cells was determined by FACS analysis using eFluor^{™}670 dilution as readout. To determine background proliferation eFluor^{™}670 dilution was determined among CD3⁺ T cells cultured with rhIL-2 but in the absence of SK-MEL-28 cells (bottom panel).
   **B)** Exemplary dot plots showing eFluor^{™} 670 dilution and the percentages of eFluor^{™} 670^{low} CD8⁺ and CD8⁻ CD3⁺ T cells of SK-MEL-28-(allo-) reactive (left) versus non-SK-MEL-28-reactive T cells (right). eFluor^{™} 670 dilution was determined after 6 days of culture.

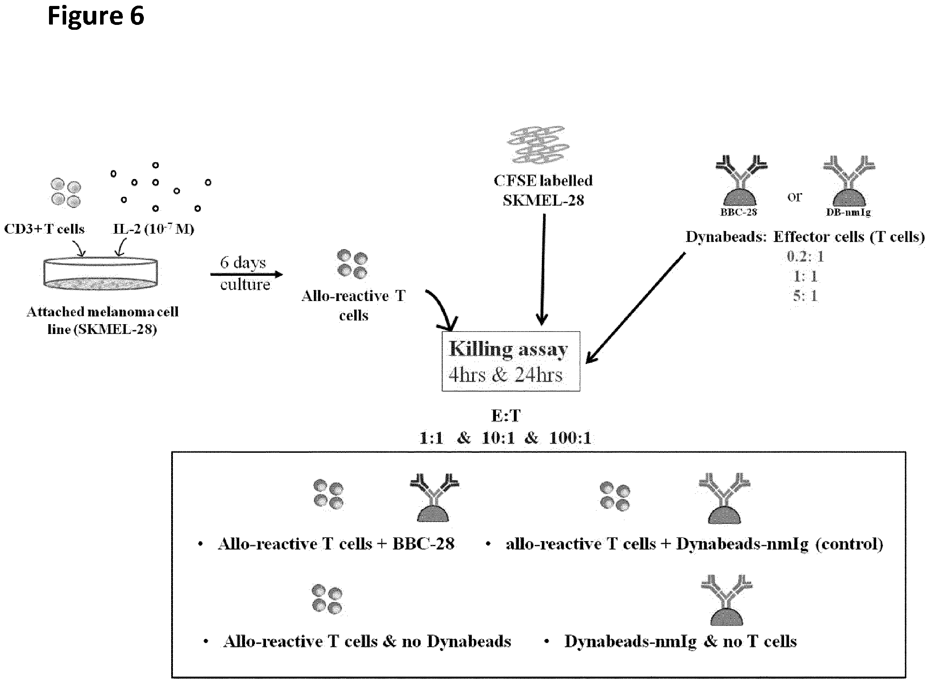

**Figure 6****.** *Anti-melanoma cell line (SK-MEL-28) 2D killing assay* - *experimental set-up.* Anti-melanoma cell line-reactive T cell generation: Isolated T cells were co-cultured with pre-seeded and Mitomycin C-treated melanoma cell line cells in the presence of rhIL-2 (10⁻⁷ M). After 6 days of co-culture, the SK-MEL-28-primed T cells were used in 2D killing assays using three different E: T ratios (1:1; 10:1; 100:1). Fresh melanoma cells (SK-MEL-28) were labeled with CFSE and used as target cells in the killing assay. Dynabeads^{™} Pan Mouse IgG were coated with either anti-CD28 mAb (clone 15E8 - BBC-28) or nmlg as a control. SK-MEL-28 target cells were incubated with T cells and Dynabeads^{™} in the following combinations: 1) allo-reactive T cells with BBC-28; 2) allo-reactive T cells with nmlg-coated Dynabeads^{™} Pan Mouse IgG; 3) allo-reactive T cells and no Dynabeads^{™}; 4) nmlg-coated Dynabeads^{™} Pan Mouse IgG and no T cells. The effect of the different Dynabead^{™} preparations on T cell cytotoxicity was evaluated in the killing assay employing three different ratios of beads to effector T cells (0.2:1; 1:1; 5:1) for each E: T ratio. Cytotoxicity was determined after 4 and 24 h.

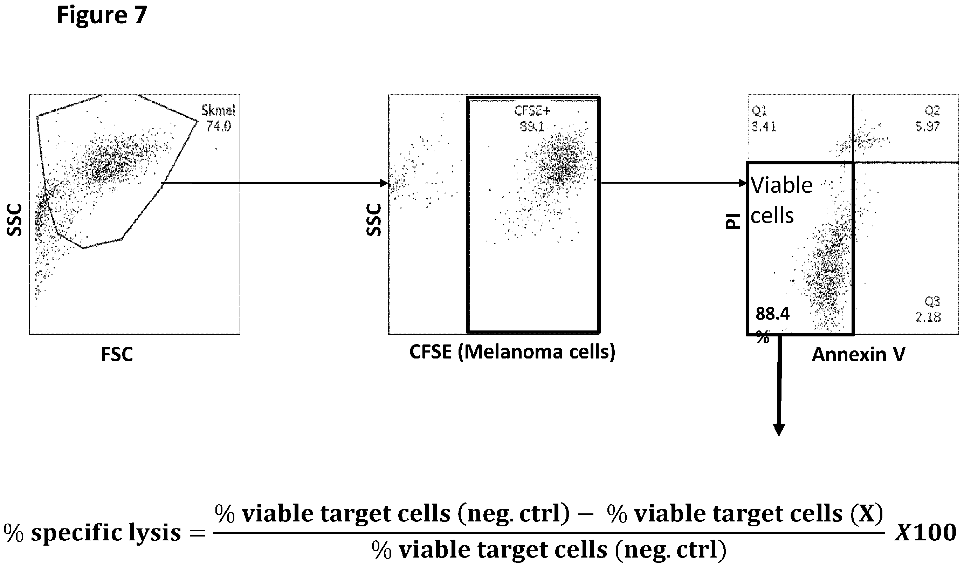

**Figure 7****.** *Gating strategy to evaluate the anti-melanoma killing effect by T cells.* Cells were first gated according to their FSC and SSC signal. SK-MEL-28 melanoma cells were identified as CFSE^{high} cells. The viability of CFSE^{high} cells was determined by plotting propidium iodide (PI) versus Annexin V. The percentage of viable melanoma cells was used to calculate the percentage of specific lysis of the cells. The percentages of specific lysis were then plotted against their respective effector: target ratios.

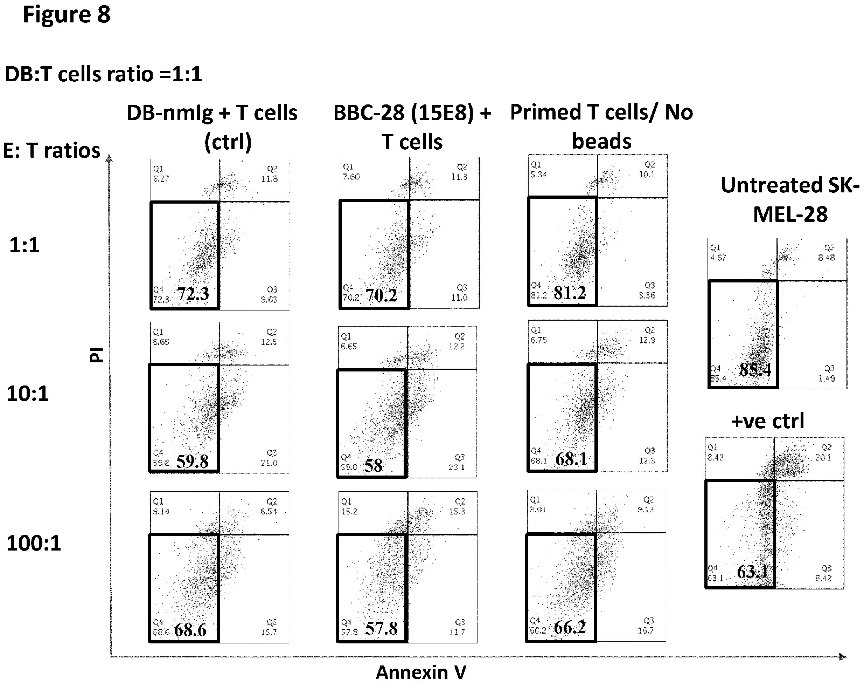

**Figure 8****.** *BBC-28 stimulation increases killing efficacy of CTLs against SK-MEL-28 cells.* 24h killing assay.
   SK-MEL-28 melanoma cells were labeled with CFSE and cultured with primed T cells at the given effector to target (E: T) ratios. Dynabead^{™} preparations were added as indicated at a Dynabead^{™} to T cell ratio as 1:1. To determine melanoma cell viability the cells were stained with propidium iodide (PI) and Annexin V. Black frames mark Annexin V⁻ PI⁻ viable (CFSE⁺) melanoma cells. Untreated melanoma cells were used to determine maximum viability. As a positive control for killing, melanoma cells were exposed to 56° C in a water bath for 10 min.
**Figure 9****.** *SK-MEL-28-primed T cells kill SK-MEL-28 target cells better in the presence of BBC-28 than in the presence of control beads.*
   Anti-SK-MEL-28 cytotoxicity is depicted as % specific lysis seen after 4 (top) and 24 h (bottom) of incubation of T cells and SK-MEL-28 cells. BBC-28 (blue) induced better killing of SK-MEL-28 cells than control (gray). Results are presented as means of % specific lysis ± SD for n= 5 donors and were analyzed by Sidak's multiple comparison test. ^{∗} p < 0.05.
**Figure 10****.** *Generation of MeWo-reactive CTLs to study specific killing of MeWo cells.* Exemplary dot plots showing the dilution and the percentage of MeWo- (allo-) reactive eFluor^{™} 670^{low} CD8⁺ and CD8⁻ CD3⁺ T cells (left) versus non-MeWo-reactive T cells (right). eFluor^{™} 670 dilution was determined after 6 days of culture.
**Figure 11****.** *The degree of allo-reactivity of T cells towards melanoma cell line cells and cytotoxic activity against MeWo melanoma cell line cells.*
   Individual percentages of eFluor^{™} 670^{low}among A) SK-MEL-28-primed and **B)** MeWo-primed CD8⁺ (left) and CD8⁻ (right) T cells in comparison to background proliferation (percentages of eFluor^{™} 670^{low} among CD8⁺ and CD8⁻ of non-primed T cells) reflecting the degree of allo-reactivity against SK-MEL-28 or MeWo cells, respectively. Results were pooled from n= 5donors for SK-MEL 28 and n=6 donors for MeWo cells and analyzed by a paired t-test, ^{∗} p < 0.05.
   C) T cells primed against MeWo melanoma cell line cells did not show enhanced killing in the presence of BBC-28 compared to control (DB-nmIg). Cytotoxicity was determined after 4 h (top) and 24 h (bottom) and is depicted as % specific lysis. Results are presented as means of % specific lysis ± SD for n= 5 donors and were analyzed by Sidak's multiple comparison test.
**Figure 12****.** *Dynabeads^{™} Human T-Activator CD3*/*CD28 are equally inefficient as BBC-28 to enhance killing of MeWo-primed CTL.*
   Results are presented as means of % specific lysis ± SD for n= 5 donors and were analyzed by Dunnett's multiple comparison test. ^{∗} p < 0.05.
**Figure 13****.** *Dynabeads^{™} are per se toxic to melanoma cell line cells in contrast to the MCC cell line PeTa.*
   **A)** Exemplary dot plots for the percentage of viable SK-MEL-28 cells (bold outlined boxes) after being cocultured with nmlg-coated Dynabeads^{™} Pan Mouse IgG (DB-nmIg) at titrated DB: target (melanoma) cell ratios.
   **B)** Toxicity of DB-nmIg against five different melanoma cell lines (A375, Malme3M, SK-MEL-28, SK-MEL-28-HLA-A2⁺ and MeWo). Toxicity tests were setup for 4 and 24 h using titrated DB: target ratios. Results from the five cell lines are presented as means of % specific lysis with individual readings for n= 3 donors.
   **C)** Means of % specific lysis comparing the effect of Dynabeads^{™} on melanoma cell line cells (MeWo and SK-MEL-28) to that on PeTa cell line cells after 4 and 24 h of co-incubation. Up to 100 Dynabeads^{™} were used per melanoma or Merkel cell carcinoma cell. Results are presented as means of % specific lysis ± SD for n= 3 donors and analyzed with a Tukey multiple comparisons test, ^{∗} p < 0.01.
**Figure 14****.** *Cytotoxicity is directly correlated to the uptake of Dynabeads^{™} by tumor cells.* Microscopic images of SK-MEL-28 and PeTa cells (original magnification: 200X and 400X respectively) after being incubated with nmlg-coated Dynabeads^{™}.SK-MEL-28 cells were found to internalize and accumulate inside the Dynabeads^{™}.This is in contrast to PeTa cells which did not internalize Dynabeads^{™}.
**Figure 15****.** *Dynabeads^{™} are widely toxic to several cancer type cells (monocytic leukemia, lung and breast adenocarcinoma in addition to melanoma cells).*
   % specific lysis graphs comparing the effect of Dynabeads^{™} on **A)** H358 and HCC44 lung adenocarcinoma cell lines; **B)** MDA-MB 231 breast adenocarcinoma and MUG-Mel 2melanoma cell lines; **C)** THP-1 monocytic leukemia cell line and the differentiated macrophages from THP-1 cell line cells to that on SK-MEL-28 cells after 4 and 24 h of co-incubation. Up to 500 Dynabeads^{™} were used per cell line cell. Results are presented as means of % specific lysis ± SD for n= 3 donors and analyzed with a Tukey multiple comparisons test, ^{∗} p < 0.01.
**Figure 16****.** *Cytotoxicity is directly correlated to the uptake of* Dynabeads^{™} *by tumor cells.* Microscopic images of the melanoma SK-MEL-28 and MUG-Mel 2, the professional phagocytes THP-1 (monocytes) and differentiated THP-1 (macrophages), the breast adenocarcinoma MDA-MB231 and the lung adenocarcinoma HCC44 and H358 cell line cells (original magnification: 200X) internalizing Dynabeads^{™}. Pictures were made usinga Leica Microsystems microscope.
**Figure 17****.** *BBC-28 delivers a double hit to melanoma cells.*
   The graph summarizes how BBC-28 are capable of activating CD4⁺ T cells to secrete cytokines which then stimulate the cytotoxic activity of CD8+ T cells against melanoma cells (first hit). In addition, uptake of BBC-28 by melanoma cells is directly toxic for the melanoma cells, thus delivering a second hit.
**Figure 18****.** *DB-nmIg and BBC-28 are equally efficient in directly inducing cytotoxicity in melanoma cells.*
   Experimental set-up was as for Figure 13. Two-way ANOVA followed by post-hoc testing corrected for multiple comparisons. ^{∗} p < 0.05, ^{∗∗} p < 0.01.
**Figure 19****.** *BBC-28 stimulation increased killing efficacy of CTLs against SK-MEL-28 cells (24 h killing assay)* - *validation experiments using nylon wool non-adherent T cells*
   For a second set of experiments using the same experimental set-up as for Figure 8 negative magnetic selection was substituted by nylon wool non-adherence to select for T cells. Also here, BBC-28 enhanced killing by primed T cells compared to DB-nmIg. Two-way ANOVA followed by post-hoc testing corrected for multiple comparisons. ^{∗∗∗∗} p < 0.0001.
**Figure 20****.** *ICB Ipilimumab enhanced CTL-mediated killing of SK-MEL-28 cells*
   Instead of BBC-28 Ipilimumab (1 µg/ ml) was added to cocultures of primed T cells and SK-MEL-28 melanoma cells. A human IgG₁ mAb (1 µg/ ml) was added as a control. Two-way ANOVA followed by post-hoc testing corrected for multiple comparisons. ^{∗} p < 0.05, ^{∗∗∗} p < 0.001.
**Figure 21****.** *The combination of anti-PD-1 and anti-PD-L1 mAb enhanced killing of melanoma cells by CTLs.*
   Instead of BBC-28, anti-PD-1 mAb Pembrolizumab and/ or anti-PD-L1 mAb Avelumab (0.5 µg/ ml each) were added during the killing assay as indicated. A human IgG₁ mAb (1 µg/ ml) was added as a control. Two-way ANOVA followed by post-hoc testing corrected for multiple comparisons. ^{∗} p < 0.05, ^{∗∗} p < 0.01.
**Figure 22****.** *BBC-28 was equally potent as the combination ofBBC-28 and PD-1*/ *PD-L1 in enhancing melanoma cell killing*
   BBC-28 and/ or anti-PD-1 mAb Pembrolizumab plus anti-PD-L1 mAb Avelumab (0.5 µg/ ml each) were added as indicated during the cytotoxicity assay. Two-way ANOVA followed by post-hoc testing corrected for multiple comparisons. ^{∗∗} p < 0.01, ^{∗∗∗} p < 0.001, ^{∗∗∗∗} p < 0.0001.

### EXAMPLES

### EXAMPLE 1 Materials and Methods

### 1.1 Pan Mouse IgG Dynabeads^{™} coating with monoclonal antibodies

Dynabeads^{™} Pan Mouse IgG (Thermo Fisher Scientific, Catalog No. 11041) were washed 3 times with PBS. Then they were coated in a concentration of 4.10⁷ cell/ ml with antibodies (15min, on ice, vortexing every 5min.). The unbound mAb were then washed out. To ensure equal coating of proteins on the beads surfaces, coated beads were incubated with 20 µg/ml nmlg (Sigma-Aldrich, Catalog No. I5381-5MG) in PBS (15min, on ice, vortexing every 5 min) and then washed 3 times with PBS.

### Anti-CD28 antibody, clone E158

- Sigma Aldrich, catalog no. CBL517 (clone 15E8, Chemicon^{®}, from mouse): https://www.sigmaaldrich.com/DE/de/product/mm/cbl517?gclid=EAIaIQobChMIwPK30aW X-wIVaI9oCR38UwKcEAAYASAAEgIjhfD_BwE&gclsrc=aw.ds. Miltenyi Biotec (catalog no. 130-093-375):https://www.miltenyibiotec.com/DE-en/products/cd28-antibody-anti-human-15e8.html#gref.

### 1.2 Cell line-reactive T cell generation in an allogeneic mixed lymphocyte reaction (allo-MLR)

Allo-reactive/ cell line-primed T cells were generated by coculturing isolated CD3⁺ T cells (Pan T cell isolation kit-Milteny Biotec or nylon wool non-adherent cells) with the intended pre-seeded and proliferation impaired (by Mitomycin C) cell line cells for 6 days in the presence of recombinant human IL-2 (rhIL-2) (Figure 5A). T cells were either labeled with eFluor 670^{™} (Thermo Fisher Scientific, Catalog No. 65-0840-85) to monitor their allo-reactivity or unlabelled to be used in the subsequent killing assays. Allo-reactivity was measured as eFluor 670^{™} dye dilutions as a reflection for % of T cell responding to the allo-stimulant.

### 1.3 Killing assay

Killing assays were performed to determine the cytotoxic abilities of BBC-28 stimulated and primed T cells. For this purpose, cell line-primed T cells were generated 6 days before the due date of killing assay (Figure 5A). At day 6, primed T cells were coincubated with fresh CFSE labeled tumor cell line cells for 4 and 24 h (Figure 6). Killing assays involved the following conditions: 1) allo-reactive T cells with BBC-28; 2) allo-reactive T cells with nmlg-coated Dynabeads^{™} (DB-nmIg); 3) allo-reactive T cells and no Dynabeads^{™}; 4) DB-nmIg and no T cells. Three effector: target (E:T) ratios (1:1, 10:1 and 100:1) were run alternatively with three Dynabeads^{™}: T cell ratios (0.2:1, 1:1 and 5:1).

### 1.4 Toxicity test

In Dynabeads^{™} toxicity investigations, Dynabeads^{™} were coated with nmlg (20 µg/ml) and co-incubated for 4 and 24h with CFSE labeled cell line cells. Dynabeads^{™}: target cell ratios ranging from 0.2:1- 500:1 were tested.

### 1.5 Specific killing measurement

The viability of CFSE^{high} gated target cells was evaluated by Annexin V and propidium iodide staining. The % specific lysis was calculated and plotted against Dynabeads^{™}: target ratios (Figure 7).

### 1.6 Statistical analysis

Summary graph and statistical testing were done using GraphPad Prism 8. P values of less than 0.05 were considered as statistically significant (^{∗}p < 0.05, ^{∗∗}p < 0.01, ^{∗∗∗}p < 0.001, ^{∗∗∗∗}p < 0.0001). Two-way-ANOVA was followed by a post-hoc test correcting for multiple comparisons (Tukey, Sidak).

The features disclosed in the foregoing description, in the claims and/or in the accompanying drawings may, both separately and in any combination thereof, be material for realizing the invention in diverse forms thereof.

### REFERENCES

Adams AB, Ford ML, Larsen CP. Costimulation Blockade in Autoimmunity and Transplantation: The CD28 Pathway. J Immunol. 2016 Sep 1;197(6):2045-50.
Alspach E, Lussier DM, Miceli AP, Kizhvatov I, DuPage M, Luoma AM, et al. MHC-II neoantigens shape tumor immunity and response toimmunotherapy. Nature. 2019 Oct 31;574(7780):696.
Azuma et al., 1992, The Journal of Experimental Medicine 175:353.
Beyersdorf N, Gaupp S, Balbach K, Schmidt J, Toyka K V., Lin CH, et al. Selective targeting of regulatory T cells with CD28 superagonists allows effective therapy of experimental autoimmune encephalomyelitis. J Exp Med. 2005 Aug 1;202(3):445.
Beyersdorf N, Hanke T, Kerkau T, Hünig T. Superagonistic anti-CD28 antibodies: potent activators of regulatory T cells for the therapy of autoimmune diseases Ann Rheum Dis. 2005 Nov;64 Suppl 4(Suppl 4):iv91-5. doi: 10.1136/ard.2005.042564.
Bischof A, Hara T, Lin C-H, Beyers AD, Hünig T. Autonomous induction of proliferation, JNK and NF-B B activation in primary resting T cells by mobilized CD28. Eur J Immunol. 2000 Mar;30(3):876-82. doi: 10.1002/1521-4141(200003)30:3.
Boegel S, Löwer M, Bukur T, Sahin U, Castle J. A catalog of HLA type, HLA expression, and neo-epitope candidates in human cancer cell lines. Oncoimmunology. 2014;3(8).
Boivin WA, Cooper DM, Hiebert PR, Granville DJ. Intracellular versus extracellular granzyme B in immunity and disease: challenging the dogma. Lab Investig 2009 8911. 2009 Sep 21;89(11):1195-220.
Castro F, Cardoso AP, Gonçalves RM, Serre K, Oliveira MJ. Interferon-Gamma at the Crossroads of Tumor Immune Surveillance or Evasion. Front Immunol. 2018 May 4;0(MAY):847.
Chen L, Flies DB. Molecular mechanisms of T cell co-stimulation and co-inhibition. Vol. 13, Nature Reviews Immunology. Nature Publishing Group; 2013. p. 227-42.
Cole, et al., 1985, in Monoclonal Antibodies and Cancer Therapy, Alan R. Liss, Inc., pp. 77-96).
Coligan et al., Current Protocols In Immunology 1992, Section 2.5.1-2.6.7.
Curtsinger JM, Mescher MF. Inflammatory Cytokines as a Third Signal for T Cell Activation. Curr Opin Immunol. 2010 Jun;22(3):333.
Del Monte U. Does the cell number 10(9) still really fit one gram of tumor tissue? Cell Cycle. 2009 Feb 1;8(3):505-6.
Dennehy KM, Kerstan A, Bischof A, Park J, Na S, Hünig T. Mitogenic signals through CD28 activate the protein kinase Cθ-NF-κB pathway in primary peripheral T cells. Int Immunol. 2003 May 1;15(5):655-63.
Dennehy KM, Elias F, Na S-Y, Fischer KD, Hünig T, Lühder F. Mitogenic CD28 signals require the exchange factor Vav1 to enhance TCR signaling at the SLP-76-Vav-Itk signalosome. J Immunol. 2007 Feb 1;178(3):1363-71. doi: 10.4049/jimmunol.178.3.1363.
Eastwood D, Findlay L, Poole S, Bird C, Wadhwa M, Moore M, et al. Monoclonal antibody TGN1412 trial failure explained by species differences in CD28 expression on CD4+ effector memory T-cells. Br J Pharmacol. 2010 Oct;161(3):512.
Harlow et al., Antibodies: A Laboratory Manual, page 726 (Cold Spring Harbor Pub. 1988).
Hombach AA, Kofler D, Rappl G, Abken H. Redirecting human CD4+CD25+ regulatory T cells from the peripheral blood with pre-defined target specificity. Gene Ther 2009 Sep;16(9):1088-96.
Jiang W, He Y, He W, Wu G, Zhou X, Sheng Q, et al. Exhausted CD8+T Cells in the Tumor Immune Microenvironment: New Pathways to Therapy. Front Immunol. 2021 Feb 2;11.
Kamata M, Tada Y. A Literature Review of Real-World Effectiveness and Safety of Dupilumab for Atopic Dermatitis. JID Innov. 2021 Sep 1;1(3):100042.
Kang K, Xie F, Mao J, Bai Y, Wang X. Significance of Tumor Mutation Burden in Immune Infiltration and Prognosis in Cutaneous Melanoma. Front Oncol. 2020 Sep 18;10:1801.
Kim MJ, Hong KH, Lee BR, H S-J. Establishment of a mechanism-based in vitro coculture assay for evaluating the efficacy of immune checkpoint inhibitors. Cancer Immunol Immunother. 2022 Nov;71(11):2777-2789. doi: 10.1007/s00262-022-03201-9. PMID: 35437609.
Kohler and Milstein, 1975, Nature, 256:495-497.
Kozbor et al., 1983, Immunology Today 4:72.
La-Beck NM, Islam MR, Markiewski MM. Nanoparticle-Induced Complement Activation: Implications for Cancer Nanomedicine. Front Immunol. 2020 Jan 8;11.
Lu X, Jian X, Liu R, Zhao H, Liang, Z. Adoptive transfer of pTRP2-specific CTLs expanding by bead-based artificial antigen-presenting cells mediates anti-melanoma response. Cancer Lett. 2008 Nov 18;271(1): 129-39. doi: 10.1016/j.canlet.2008.05.049.
Lugini L, Lozupone F, Matarrese P, Funaro C, Luciani F, Malorni W, et al. Potent phagocytic activity discriminates metastatic and primary human malignant melanomas: a key role of ezrin. Lab Invest. 2003 Nov;83(11):1555-67.
Luhder, F., Huang, Y., Dennehy, K.M., Guntermann, C, Muller, I., Winkler, E., Kerkau, T., Ikemizu, S., Davis, S.J., Hanke, T., et al. 2003. Topological requirements and signaling properties of T cell-activating, anti-CD28 antibody superagonists. J Exp Med 197:955-966.
Nowicki TS, Hu-Lieskovan S, Ribas A. Mechanisms of Resistance to PD-1 and PD-L1 blockade. Cancer J. 2018 Jan 1;24(1):47.
Nunes et al., 1993, International Immunology 5:11.
Ostrov DA, Shi W, Schwartz JC, Almo SC, Nathenson SG Structure of murine CTLA-4 and its role in modulating T cell responsiveness. Science (2000), 290:816-819.
Platanias LC. Mechanisms of type-I- and type-II-interferon-mediated signalling. Nat Rev Immunol 2005 55. 2005 May;5(5):375-86.
Pohl C, Denfeld R, Renner C, Jung W, Bohlen H, Sahin U, Hombach A, van Lier R, Schwonzen M, Diehl V, Pfreundschuh M. CD30-antigen-specific targeting and activation of T cells via murine bispecific monoclonal antibodies against CD3 and CD28: potential use for the treatment of Hodgkin's lymphoma. Int J Cancer 1993 Jul 9;54(5):820-7.
Poirier N, Blancho G, Vanhove B. CD28-Specific Immunomodulating Antibodies: What Can Be Learned From Experimental Models? Am J Transplant. 2012 Jul 1;12(7):1682-90.
Ribas A, Wolchok JD. Cancer immunotherapy using checkpoint blockade. Science (80-). 2018 Mar 23;359(6382): 1350-5.
Römer PS, Berr S, Avota E, Na SY, Battaglia M, Ten Berge I, et al. Preculture of PBMCs at high cell density increases sensitivity of T-cell responses, revealing cytokine release by CD28 superagonist TGN1412. Blood. 2011 Dec 22;118(26):6772-82.
Rosskopf S, Leitner J, Zlabinger GJ, Steinberger P. CTLA-4 antibody ipilimumab negatively affects CD4+ T-cell responses in vitro. Cancer Immunol Immunother. 2019 Aug 22;68(8):1359-68.
Sanchez-Lockhart M, Rojas A V., Fettis MM, Bauserman R, Higa TR, Miao H, et al. T Cell Receptor Signaling Can Directly Enhance the Avidity of CD28 Ligand Binding. PLoS One. 2014 Feb 24;9(2):89263.
Schrama D, Sarosi EM, Adam C, Ritter C, Kaemmerer U, Klopocki E, et al. Characterization of six Merkel cell polyomavirus-positive Merkel cell carcinoma cell lines: Integration pattern suggest that large T antigen truncating events occur before or during integration. Int J Cancer. 2019 Aug 15;145(4):1020-32.
Schummer P, Schilling B, Gesierich A. Long-Term Outcomes in BRAF-Mutated Melanoma Treated with Combined Targeted Therapy or Immune Checkpoint Blockade: Are We Approaching a True Cure? Am J Clin Dermatol. 2020 Aug 1;21(4):493-504.
Sheth RA, Sabir S, Krishnamurthy S, Avery RK, Zhang YS, Khademhosseini A, et al. Endovascular Embolization by Transcatheter Delivery of Particles: Past, Present, and Future. J Funct Biomater. 2017 Apr 3;8(2):12.
Smith-Garvin JE, Koretzky GA, Jordan MS. T cell activation. Vol. 27, Annual Review of Immunology. Annu Rev Immunol; 2009. p. 591-619.
Suntharalingam G, Perry MR, Ward S, Brett SJ, Castello-Cortes A, Brunner MD, et al. Cytokine Storm in a Phase 1 Trial of the Anti-CD28 Monoclonal Antibody TGN1412. https://doi.org/101056/NEJMoa063842. 2009 Oct 8;355(10):1018-28.
Takemoto M, Asker N, Gerhardt H, Lundkvist A, Johansson BR, Saito Y, et al. A New Method for Large Scale Isolation of Kidney Glomeruli from Mice. Am J Pathol. 2002;161(3):799.
Tan P, Anasetti C, Hansen JA, Melrose J, Brunvand M, Bradshaw J, et al. Induction of alloantigen-specific hyporesponsiveness in human T lymphocytes by blocking interaction of CD28 with its natural ligand B7/BB1. J Exp Med. 1993 Jan 1;177(1):165.
Ugel S, Zoso A, De Santo C, Li Y, Marigo I, Zanovello P, et al. In vivo administration of artificial Antigen Presenting Cells activates low avidity T cells for treatment of cancer. Cancer Res. 2009 Dec 15;69(24):9376.
Uribe-Querol E, Rosales C. Phagocytosis: Our Current Understanding of a Universal Biological Process. Front Immunol. 2020 Jun 2;11:1066.
Vanhove B, Poirier N, Fakhouri F, Laurent L, Hart B 't, Papotto PH, et al. Antagonist Anti-CD28 Therapeutics for the Treatment of Autoimmune Disorders. Antibodies. 2017 Nov 21;6(4):19.
Van Lier RA, Brouwer M, De Jong R, Groot M, Groot E, Arden LA. Functional properties of the human T cell differentiation antigen CD28. In: W. Knapp et al. (eds.), Leukocyte Typing IV, pp. 353 - 355, Oxford University Press, Oxford (1989).
Wei SC, Duffy CR, Allison JP. Fundamental mechanisms of immune checkpoint blockade therapy. Vol. 8, Cancer Discovery. American Association for Cancer Research Inc.; 2018. p. 1069-86.
WHO. Melanoma of skin Source: Globocan 2020. Available from: https://gco.iarc.fr/today.
WHO. Cancer Today 2020. Available from: https://gco.iarc.fr/today/online-analysis-map?v=2020&mode=population&mode_population=continents&population=900&population s=900&key=asr&sex=0&cancer=39&type=0&statistic=5&prevalence=0&population_group= 0&ages_group%5B%5D=0&ages_group%5B%5D=17&nb_items=10&gr.
Wu X, Gu Z, Chen Y, Chen B, Chen W, Weng L, et al. Application of PD-1 Blockade in Cancer Immunotherapy. Vol. 17, Computational and Structural Biotechnology Journal. Elsevier B.V.; 2019. p. 661-74.
Yeung SJ, Qdaisat A, Chaftari P, Lipe D, Merlin J, Rajha E, et al. Diagnosis and management of immune-related adverse effects of immune checkpoint therapy in the emergency department. J Am Coll Emerg Physicians Open. 2020 Dec; 1(6): 1637.

## Claims

1. A solid microbead having an anti-CD28 monoclonal antibody immobilized thereto for use in the local treatment of malignant melanoma,
wherein the anti-CD28 monoclonal antibody binds to an epitope on CD28 outside the C"D loop.

2. The solid microbead for use according to claim 1, wherein the anti-CD28 monoclonal antibody is a conventional anti-CD28 monoclonal antibody,
and/or wherein the anti-CD28 monoclonal antibody is clone 15E8, CD28.2 or L293.

3. The solid microbead for use according to claim 1 or 2, wherein the anti-CD28 monoclonal antibody is not a super-agonistic anti-CD28 monoclonal antibody.

4. The solid microbead for use according to any one of claims 1 to 3, wherein the anti-CD28 monoclonal antibody is the only active ligand immobilized onto the bead,
and/or wherein the bead does not have any further anti-CD antibodies immobilized thereto.

5. The solid microbead for use according to any one of claims 1 to 4, wherein the malignant melanoma is metastatic melanoma.

6. The solid microbead for use according to any one of claims 1 to 5, wherein the local treatment comprises intradermal, intralesional and subcutaneous injection,
preferably injection into the metastases and the border zone of metastasis and healthy skin, and/or wherein intralesional administration preferably comprises image-guided injection into visceral metastases.

7. The solid microbead for use according to any one of claims 1 to 6, wherein the microbead has a diameter in the range of about 0,5 µm to about 10 µm, preferably 4 to 5 µm.

8. The solid microbead for use according to any one of claims 1 to 7, wherein the microbead is a magnetic beat, preferably a superparamagnetic polystyrene bead.

9. The solid microbead for use according to any one of claims 1 to 8, wherein the anti-CD28 monoclonal antibody is indirectly immobilized to the microbead, preferably via an antibody coated onto the bead,
wherein the antibody coated onto the bead is preferably a monoclonal or polyclonal human anti-mouse Ig antibody.

10. The solid microbead for use according to any one of claims 1 to 9, wherein the solid microbeads are mixed with autologous T cells for intralesional administration, preferably at a ratio of microbeads to T cell of about 5:1.

11. The solid microbead for use according to any one of claims 1 to 10, wherein the use is in combination with one or more further therapies for malignant melanoma, preferably for metastasizing melanoma.

12. The solid microbead for use according to claim 11, wherein the one or more further therapies is:
- immunotherapy, such as with checkpoint inhibitors,
- targeted therapy, such as with BRAF inhibitors or MEK inhibitors,
- chemotherapy,
- radiotherapy,
- surgical treatment,
or combinations thereof.

13. A method for the local treatment of malignant melanoma comprising the step of
administering a therapeutically effective amount of solid microbeads as defined in any one of claims 1 to 4 and 7 to 9, or
administering a pharmaceutical composition comprising the solid microbeads as defined in any one of claims 1 to 4 and 7 to 9,
preferably via intradermal, intralesional or subcutaneous injection,
wherein the malignant melanoma is preferably metastasizing melanoma.

14. The method of claim 13, wherein the local treatment comprises injection into the metastases and the border zone of metastasis and healthy skin, and/or wherein intralesional administration comprises image-guided injection into visceral metastases,
and/or wherein the solid microbeads are mixed with autologous T cells for intralesional administration, preferably at a ratio of microbeads to T cell of about 5:1.

15. The method of claim 13 or 14 in combination with one or more further therapies for malignant melanoma, preferably for metastatic melanoma,
wherein the one or more further therapies is/are preferably:
- immunotherapy, such as with checkpoint inhibitors,
- targeted therapy, such as with BRAF inhibitors or MEK inhibitors,
- chemotherapy,
- radiotherapy,
- surgical treatment,
or combinations thereof.
